# EUROPEAN PATENT SPECIFICATION

(11) **EP 3 723 625 B1**
(45) Date of publication and mention of the grant of the patent: **23.10.2024**
(21) Application number: 18888374.8
(22) Date of filing: 12.12.2018
(51) Int. Cl.: A61B 17/04, A61B 17/062

(54) **SUTURING END-EFFECTOR**
ENDEFFEKTOR FÜR NAHTMATERIAL
EFFECTEUR TERMINAL DE SUTURE

(30) Priority: 12.12.2017 SG 10201710343U
(43) Date of publication of application: 21.10.2020
(73) Proprietor: Nanyang Technological University, Singapore 639798 (SG)
(72) Inventor: CAO, Lin, Singapore 639798 (SG); PHAN, Phuoc Thien, Singapore 639798 (SG); PHEE, Soo Jay Louis, Singapore 639798 (SG)
(74) Representative: Viering, Jentschura & Partner mbB Patent- und Rechtsanwälte
(86) International application number: PCT/SG2018/050606
(87) International publication number: WO 2019/117811

(56) References cited:
- EP-A2- 2 462 877
- WO-A1-2006/122764
- WO-A1-2017/044838
- WO-A1-2017/155406
- US-A- 5 569 301
- US-A1- 2015 142 018
- US-B2- 8 747 424
- US-B2- 8 968 339

## Description

### Cross-reference to Related Applications

The present application claims the benefit of the Singapore patent application No. 10201710343U filed on 12 December 2017.

### Technical Field

Various embodiments generally relate to a suturing end-effector. In particular, various embodiments generally relate to a suturing end-effector for an endoscopic instrument that may be used to perform endoscopic closure.

### Background

Natural Orifice Transluminal Endoscopic Surgery (NOTES) is an endoscopic surgical intervention technique for treatments within the intraperitoneal cavity such as the stomach and colon through natural orifices, e.g., mouth, vagina, and anus. Compared to open surgery and laparoscopic surgery, NOTES avoids incisions on abdominal wall and thus are less invasive, safer, and allow patients to recover faster. Although techniques for NOTES were greatly advanced in recent years, the full potential of NOTES is not possible without reliable and safe closure of defects. These defects can be caused by un-planned complications (perforation) of diagnostic and therapeutic endoscopy or by the full-thickness resection of gastrointestinal tract (GI tract) lesions or by planned perforations for NOTES access. Endoscopic closure of GI tract defects is challenging due to the limited sizes of the endoscope channel and the GI tract, the confined space at the target sites, the requirements on the size and strength of wound closure, and the complexity of suturing/closure task itself. Over the years, various instruments and accessories have been configured for the closure of GI tract defects. However, each configuration has its inherent problems that need to be accommodated during the surgery. For instance, for the commercially available Over-The-Scope-Clip system (Ovesco Endoscopy, Inc., Germany) and Overstitch Endoscopic Suturing System (Appollo Endosurgery, Inc., US), the end-effectors have to be preloaded to the outer-tip of the endoscope and thus the endoscope has to be withdrawn to change tools.

Further, US patent no. US 8,747,424 B2 discloses a suturing instrument with flexible transmission system and thus maybe suitable for endoscopy. A flexible actuation shaft is employed to actuate two jaws and a locking blade in each jaw, and another pair of cables is employed to steer the end-effector for triangulation. By pulling (or pushing) the actuation shaft, the two jaws can be closed (or open). By twisting the actuation shaft (torsion), the locking blades can be moved/translated along the longitudinal axis of the instrument for locking the suturing needle to either of the jaws. This is achieved through a camming hub which transfers the torsion of the actuation shaft to translational motion. In this way, the suturing needle can be switched from one jaw to the other. However, the instrument as disclosed in said patent has several shortcomings. Firstly, the outer diameter of the instrument of said patent is rather large because the suturing needle is always standing erected in between the two jaws causing the jaws to always be in an ajar configuration when closed, and thus the outer diameter of the device has to be larger or equal to the length of the needle (6 ~ 10 mm long), which makes it impossible to be delivered to the surgical site through the tool channels of the endoscope in practice. Secondly, the force and motion transmission mechanisms of the instrument of said patent for the locking blades are not reliable and not efficient. The locking blades are translated along the longitudinal axis by twisting the proximal end of the flexible shaft whose distal end is connected to a torsion-to-translation camming mechanism. Torsion transmission is not reliable, if not possible, in endoscopy where the path of the GI tract is tortuous and long. The reliability of the locking blades is highly limited by the low transmission efficiency and accuracy of torsion from the proximal end to the distal end of the flexible shaft and the efficiency of the camming mechanism (torsion to translation). In addition, since the opening of the jaws are achieved by pushing the flexible shaft, the jaws may be difficult to be open in some cases because the flexible shaft may not be good at transferring a pushing force to effect the opening of the jaws. Thirdly, the instrument of said patent has only two cables employed for steering, resulting in the instrument being steerable in only one plane, limiting its dexterity and usefulness.

EP 2462877 A2 discloses a surgical apparatus for use in endoscopic and/or laparoscopic procedures. The end effector 100 of the surgical apparatus includes a first jaw member 110 and a second jaw member 120, wherein the second jaw member 120 has a split nest slide 310 coupled thereto to position a needle 400 in a folded or unfolded position. However, setting up of the needle 400 in the second jaw member 120 requires several steps. For example, (i) the movable needle blade 322 has to be aligned to the aperture 312 of the movable split nest slide 310 for inserting the needle, (ii) after the needle 400 is inserted, the needle blade 322 is pulled so that channel 428 of needle 400 slides between fingers of needle blade 322 while channel 430 of needle 400 slides between opposing edges of aperture 312 of split nest slide 310, (iii) the aperture 326 of flexible link 324 is aligned to a notch 314 of the split nest slide 310, and (iv) pin 320 is inserted via the notch 314 of the split nest slide 310 through channel 124a on one side of the second jaw member 120, through aperture 326 and out the other side of the second jaw member 120 through a corresponding channel 124b.

US 2015/0142018 A1 discloses several needles for use with an endoscopic stitching device. In one embodiments, the first and second end portions of the needle are pivotable relative to each other. In another embodiment, the needle includes two separate parts which have to be aligned and joined together when the needle is at the surgical site. In yet another embodiment, the first and second end portions of the needle are slidably translatable via a compressible element.

Accordingly, there is a need for a reliable, effective, and efficient tool for the endoscopic closure of GI tract defects.

### Summary

According to various embodiments, there is provided a suturing end-effector (or a suturing device having a suturing end-effector). The suturing end-effector includes a jaw assembly including a first jaw having a through-hole which is disposed at an end portion of the first jaw and which extends through the first jaw from an engagement surface of the first jaw to an opposite surface of the first jaw, and a second jaw having a through-hole which is disposed at an end portion of the second jaw and which extends through the second jaw from an engagement surface of the second jaw to an opposite surface of the second jaw. The first jaw and the second jaw are openable and closeable relative to each other with the engagement surface of the first jaw and the engagement surface of the second jaw directed towards each other. The suturing end-effector further includes a suture needle assembly including a hollow suture needle having a first pointed tip, a second pointed tip and an inner channel extending through the hollow suture needle from the first pointed tip to the second pointed tip. In a stowed disposition of the suturing end-effector, the hollow suture needle is clamped flat between the engagement surface of the first jaw and the engagement surface of the second jaw, and a guide wire runs through the through-hole of the first jaw, the inner channel of the hollow suture needle, and the through-hole of the second jaw in a manner such that opening the first jaw and the second jaw relative to each other and tensioning the guide wire cause the guide wire to align the hollow suture needle with the first pointed tip of the hollow suture needle directed towards the through-hole of the first jaw and the second pointed tip of the hollow suture needle directed towards the through-hole of the second jaw.

### Brief description of the drawings

In the drawings, like reference characters generally refer to the same parts throughout the different views. The drawings are not necessarily to scale, emphasis instead generally being placed upon illustrating the principles of the invention. In the following description, various embodiments are described with reference to the following drawings, in which:
FIG. 1A to FIG. 1D show schematic diagrams of a suturing end-effector according to various embodiments;
FIG. 2A to FIG. 2K show a suturing device having a suturing end-effector according to various embodiments;
FIG. 3A to FIG. 3D shows a suture needle assembly according to various embodiments;
FIG. 4A shows a cross-sectional side view, a right side view, a front view, a left side view and a perspective front-left view of a hollow suture needle according to various embodiments;
FIG. 4B shows a cross-sectional view of the hollow suture needle in a needle hole according to various embodiments;
FIG. 5A and FIG. 5B show cut-out view of the lower jaw of the suturing end-effector according to various embodiments;
FIG. 6 shows a transparent view of the suturing device of FIG. 2A to FIG. 2K according to various embodiments;
FIG. 7A and FIG. 7B show schematic diagrams of a tendon and pulley locking mechanism for the suturing device according to various embodiments;
FIG. 7C show schematic diagrams of a tendon and pulley locking mechanism for the suturing device according to various embodiments;
FIG. 8A and FIG. 8B show the tendon and pulley locking mechanism applied in the suturing end-effector according to various embodiments;
FIG. 9A to FIG. 9C show the locking blades movement for the switching of the hollow suture needle between jaws according to various embodiments;
FIG. 10 shows the routing of tendons for the locking blades where the jaws, joint links, and sheaths are removed to aid viewing the tendons according to various embodiments;
FIG. 11A shows a cut out view of a proximal end of the first jaw and FIG. 11B shows a cross-section view of the first jaw according to various embodiments;
FIG. 12A to FIG. 12C show an approach to attaching the locking blade to the tendon according to various embodiments;
FIG. 13A and FIG. 13B shows the suturing device according to various embodiments;
FIG. 14 shows an exploded view of the suturing device according to various embodiments;
FIG. 15A to FIG. 15C depicts how the tendons are connected to a grasper having the jaws according to various embodiments;
FIG. 16A to FIG. 16C show another pair of tendons may be employed to steer the first jaw relative to the first joint link according to various embodiments;
FIG. 17A and FIG. 17B show a perspective view and a broken-out section view of the first joint link according to various embodiments;
FIG. 18 shows a front view, a top view, a bottom view and a left side view of the first joint link according to various embodiments;
FIG. 19A and FIG. 19B show a further pair of tendons may be employed to steer the first joint link relative to the second joint link according to various embodiments;
FIG. 20 shows a broken-out section view of the second joint link according to various embodiments;
FIG. 21 shows a front view, a top view and a left view of the second joint link according to various embodiments;
FIG. 22 shows a bonding between the shaft body and the second joint link according to various embodiments; and
FIG. 23A to FIG. 23E show a suturing process using the suturing device according to various embodiments.

### Detailed description

Embodiments described below in the context of the apparatus are analogously valid for the respective methods, and vice versa. The methods disclosed herein do not form part of the invention. Furthermore, it will be understood that the embodiments described below may be combined, for example, a part of one embodiment may be combined with a part of another embodiment.

It should be understood that the terms "on", "over", "top", "bottom", "down", "side", "back", "left", "right", "front", "lateral", "side", "up", "down" etc., when used in the following description are used for convenience and to aid understanding of relative positions or directions, and not intended to limit the orientation of any device, or structure or any part of any device or structure. In addition, the singular terms "a", "an", and "the" include plural references unless context clearly indicates otherwise. Similarly, the word "or" is intended to include "and" unless the context clearly indicates otherwise.

Various embodiments generally relate to a suturing end-effector. In particular, various embodiments generally relate to a suturing end-effector for an endoscopic instrument that may be used to perform endoscopic closure. Various embodiments may be a through-the-scope multi-degrees-of-freedom endoscopic suturing end-effector. According to various embodiments, the suturing end-effector may be part of a surgical suturing apparatus or a surgical endoscopic suturing device. In other words, various embodiments have also provided a device having the suturing end-effector of the various embodiments. According to various embodiments, the suturing end-effector and/or the device may efficiently perform endoscopic closure for a gastrointestinal tract (GI tract), featuring with triangulation capability and fast change of tools during operations. According to various embodiments, the surgical suturing apparatus (or the surgical endoscopic suturing device or the device) may include a flexible tubular body shaft, an articulated joint assembly extending from the body shaft for articulation in directions transverse to a longitudinal axis of the assembly, two jaws pivotally connected to the articulated joint assembly, a suture needle operatively associated with the two jaws through respective needle-receiving-hole in each jaw, two movable locking blades in the jaws to lock or unlock the suture needle relative to the jaws, and tendon-sheath mechanisms for the actuation of the joints of the articulated joint assembly, the two jaws, and the two locking blades. According to various embodiments, the approach to locking or unlocking the suture needle by moving the locking blades through a tendon-sheath mechanism may be provided. In addition, various embodiments may have two modes of operation: a suturing mode and a transportation mode. The suturing mode may be the mode when the suturing end-effector and/or the device according to the various embodiments is performing suturing tasks or ready for suturing tasks. The transportation mode may be the mode when the suturing end-effector and/or the device according to the various embodiments is being transported or ready to be transported through the tool channel of the endoscope. Further, various embodiments have provided an approach to reconfigure the suturing end-effector between the two modes.

According to various embodiments, the the suturing end-effector and/or the device may be a 'Through-the-Scope' device, whereby the suturing end-effector and/or the device may be delivered to the surgical target through the tool channel of the endoscope. 'Through-the-scope' tools may allow efficient tool changes during operations. According to various embodiments, the suturing end-effector and/or the device may perform running stitches and interrupted stitches. According to various embodiments, the suturing end-effector and/or the device may allow 'Triangulation' whereby the suturing end-effector and/or the device may have the degrees of freedom for the triangulation by itself instead of relying on the maneuverability of the endoscope. Triangulation on its own may improve dexterity and ease of use of the suturing end-effector and/or the device. According to various embodiments, the suturing end-effector and/or the device may minimise or reduce the risk of puncturing other organs adjacent to the surgical site. According to various embodiments, the suturing end-effector and/or the device may perform full-thickness closure of the GI tract. Full-thickness closure may allow a wide range of surgical procedures to be performed and also improve strength of closure. According to various embodiments, the suturing end-effector and/or the device may perform closure for relatively large defects (length >=2 cm). Closure of large defects may allow for wider range of surgical procedures.

Compared to the conventional devices, various embodiments may have the following novelties.

Various embodiments have provided a suturing end-effector and/or a device features with two modes: a transportation mode and a suturing mode. According to various embodiments, the outer diameter of the suturing end-effector and/or the device in transportation mode may be significantly smaller than that in the suturing mode. Thus, various embodiments in the transportation mode may be small enough to be delivered to the surgical site through the tool channel of the endoscope. Once reaching the surgical site, the suturing end-effector and/or the device according to the various embodiments may be converted to the suturing mode, and it may be large enough to perform full-thickness suturing for GI defects. In conventional suturing devices, there is no such a transportation mode and the outer diameter of conventional suturing devices is usually too large for the channels of the endoscope. Further, various embodiments may be provided with a more reliable force and motion transmission system as compared to conventional devices. The movement of the locking blades of the various embodiments may be controlled through tendons. By pulling any of the tendons, the locking blades may be moved forward or backward along a longitudinal axis of the various embodiments. In addition, according to various embodiments, the two jaws may be controlled through another two tendons. By pulling any of the other two tendons, the jaws may be opened or closed. This approach in the various embodiments may be more reliable than the approach adopted in conventional devices. Furthermore, various embodiments may be driven by flexible tendon-sheath mechanisms with multiple actuated degrees of freedom so that various embodiments do not rely on the movement of the endoscope to move from one site to the other, and the triangulation with respect to other tools such as the gripper or cautery knives may be obtained. Most conventional devices do not have articulated joints and thus rely on the movement of the endoscope.

Various embodiments have provided a suturing end-effector and/or a device that may be small enough to be delivered through the tool channel of an endoscope and may facilitate tool triangulation by having joints which are powered through tendon-sheath mechanisms. In addition, the suturing end-effector and/or the device according to various embodiments may generate continuous or disrupted stitches for full-thickness suturing of large defects without blind needle puncture. With the suturing end-effector and/or the device according to various embodiments, once stitches are formed, knots may be easily tied, owing to the dexterity provided by the actuated joints.

Compared to conventional devices, various embodiments may have the following significant advantages.

The suturing end-effector and/or the device according to the various embodiments may convert between a normal configuration (suturing mode) and a much smaller configuration (transportation mode) so that the suturing end-effector and/or the device may be delivered or withdrawn through an endoscope channel. Once reaching the surgical site, the suturing end-effector and/or the device of the various embodiments may be converted to the suturing mode such that it is large enough to perform full-thickness suturing for GI defects. However, in conventional devices, there is no such a transportation mode and the outer diameter of the end-effector of the conventional devices is usually too large to be delivered through the channels of the endoscope. Further, various embodiments may be provided with a more reliable force and motion transmission system. In various embodiments, the movement of the locking blades may be controlled through two tendons. By pulling any of the two tendons, the locking blades may be moved forward or backward along a longitudinal axis of the respective embodiments. In addition, the two jaws in the various embodiments may also be controlled through another two tendons. By pulling any of the two tendons, the jaws may be opened or closed. This approach in the various embodiments may be more reliable than the approach adopted in conventional devices. In addition, the suturing end-effector and/or the device according to various embodiments may be stronger and more stable in holding payloads than the conventional device. The joints in the various embodiments may all be actuated while the conventional device usually has too many under-actuated joints. Under-actuated joints may not be controllable and may not be stable when encountering external loads, resulting in difficulty in manipulating tissue.

According to various embodiments, a surgical endoscopic suturing device (or a surgical suturing apparatus or a suturing device or a device) may be provided with a flexible body portion and a suturing end-effector. According to various embodiments, the suturing end-effector may be provided with an articulated joint assembly extending from the flexible body portion for articulation in (one or two or more or a plurality of) directions transverse to a longitudinal axis of the suturing end-effector, two jaws pivotally connected to the articulated joint assembly, a suture needle operatively associated with the two jaws through respective needle-receiving-recess/hole in each jaw, two movable locking blades in the jaws for locking or releasing the suture needle, and (one or two or more or a plurality of) tendon-sheath mechanisms for the actuation of the articulated joints assembly, the two jaws, and the two locking blades. According to various embodiments, locking or unlocking the suture needle by moving the locking blades may be provided through a tendon-sheath mechanism.

In addition, according to various embodiments, the suturing device may have two modes: a suturing mode and a transportation mode. The suturing mode may be the mode when the suturing end-effector of the suturing device is doing the suturing task or ready for the suturing task. The transportation mode may be the mode when the suturing end-effector of the suturing device is being transported or ready to be transported through a tool channel of an endoscope. An approach may be provided to reconfigure the suturing device between these two modes. According to various embodiments, a difference between the suturing mode and the transportation mode lies in whether the suture needle is engaged with one of the two jaws of the suturing end-effector of the suturing device. In the transportation mode, the two jaws may be completely closed (or with a gap close to the outer diameter of the suture needle), and the suture needle may not be locked to any jaws but merely lies in-between the two closed jaws (or being clamped between the two closed jaws in a horizontal or lengthwise orientation). A thin wire may go through the suture needle (which may be hollow) and the two recessing holes on the jaws (or the respective needle-receiving-recess/hole), both ends of the wire may be extended to the proximal side of the suturing device and may be pulled by motors or hands. With the suture needle lying between the two jaws, the suturing device may be small enough to be delivered through the tool channel of the endoscope. After reaching the surgical site, the jaws may be opened, and the suture needle may be deployed to stand or erect in-between the two open jaws and then to engage with one of the two jaws, i.e., the suturing device turns to its suturing mode and becomes ready for the suturing task. This may be achieved by applying tension to (pulling) the thin wire that goes through the hollow suture needle and the recessing holes of the jaws. With tension on the wire, the suture needle may stand up or may be erected between the two jaws and may be guided to the recessing holes of the jaws. Then, the locking blade on either jaw may be advanced forward to engage with a recess (or a slot) on the suture needle and thus lock it into position on the jaw. After that, one end of the thin wire may be pulled to completely withdraw the wire from the endoscope, and the suturing device may now be in its suturing mode. Alternatively, to withdraw the thin guide wire, a distal segment of the guidewire which is between the two jaws may be cut using an endoscopic scissors or cutter, and then the two proximal ends of the guidewire may be pulled to withdraw the wire from the endoscope. A weak point may also be created initially at the distal segment of the guidewire so that the guidewire can be broken when a relatively larger force (compared with the force for tensioning the wire for needle deployment) is applied to pull the two proximal ends of the guidewire. By repetitively closing/opening the jaws and switching the locking blades to lock or unlock the suture needle, stitches may be made on the target tissue. Once suturing task is done, the suturing device may be switched back to the transportation mode. The suture needle may be unlocked from the receiving jaw, and another coordinating grasper or tool may be used to remove the suture from the needle hole of the jaw. Next, the two jaws of the suturing device may grasp the removed suture needle from the coordinating grasper so that the suture needle may lay in between the two closed jaws (or may be clamped between the two closed jaws in a horizontal or lengthwise orientation). Alternatively, the coordinating grasper may grip the needle and take the needle out through the tool channel of the endoscope. Finally, the suturing device may be withdrawn by pulling the proximal end of its flexible body portion through the endoscope channel.

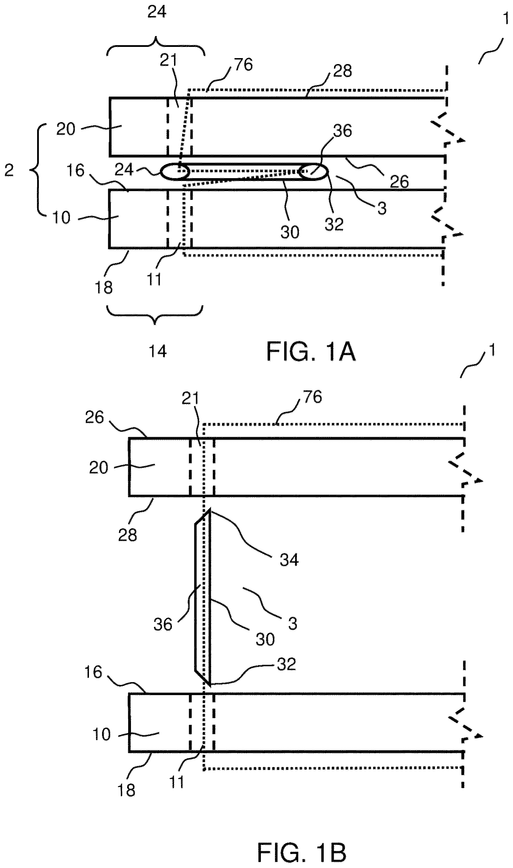

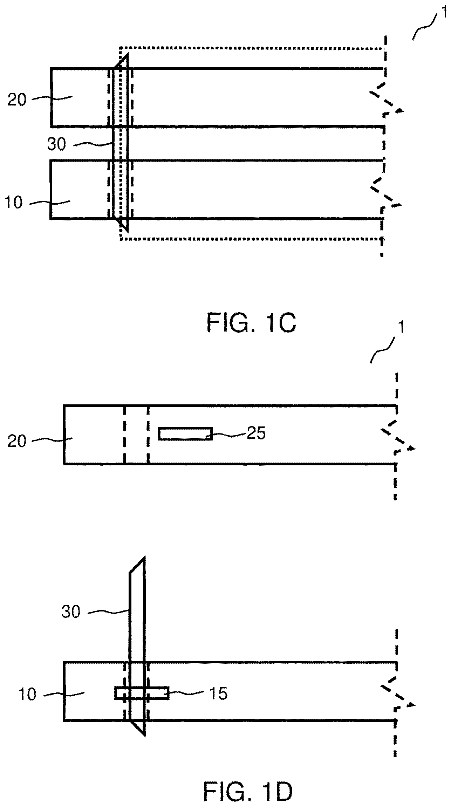

FIG. 1A to FIG. 1D show schematic diagrams of a suturing end-effector 1 according to various embodiments. FIG. 1A shows the schematic diagram of the suturing end-effector 1 in a transportation mode. FIG. 1D shows the schematic diagram of the suturing end-effector 1 in a suturing mode. FIG. 1A to FIG. 1D show the suturing end-effector 1 transforming or converting from the transportation mode to the suturing mode. According to various embodiments, the suturing end-effector 1 may be part of a suturing device. According to various embodiments, the suturing end-effector 1 includes a jaw assembly 2. The jaw assembly 2 includes a first jaw 10 (or a lower jaw) and a second jaw 20 (or an upper jaw). Each of the first jaw 10 and the second jaw 20 may have an elongate body. According to various embodiments, the first jaw 10 includes a through-hole 11 disposed at an end portion 14 of the first jaw 10. The end portion 14 of the first jaw 10 may be a free-end portion of the first jaw 10 distal to a joint or a connection (not shown) between the first jaw 10 and the second jaw 20. According to various embodiments, the through-hole 11 of the first jaw 10 extends through the first jaw 10 from an engagement surface 16 of the first jaw 10 to an opposite surface 18 of the first jaw 10. Accordingly, the through-hole 11 of the first jaw 10 may be a hole that goes all the way through the first jaw 10 from the engagement surface 16 of the first jaw 10 to the opposite surface 18 of the first jaw 10. The engagement surface 16 and the opposite surface 18 may be two surfaces on opposite sides of the first jaw 10. According to various embodiments, the second jaw 20 includes a through-hole 21 disposed at an end portion 24 of the second jaw 20. The end portion 24 of the second jaw 20 may be a free-end portion of the second jaw 20 distal to the joint or the connection between the first jaw 10 and the second jaw 20. According to various embodiments, the through-hole 21 of the second jaw 20 extends through the second jaw 20 from an engagement surface 26 of the second jaw 20 to an opposite surface 28 of the second jaw 20. Accordingly, the through-hole 21 of the second jaw 20 may be a hole that goes all the way through the second jaw 20 from the engagement surface 26 of the second jaw 20 to the opposite surface 28 of the second jaw 20. The engagement surface 26 and the opposite surface 28 may be two surfaces on opposite sides of the second jaw 20.

According to various embodiments, the first jaw 10 and the second jaw 20 are openable and closeable relative to each other with the engagement surface 16 of the first jaw 10 and the engagement surface 26 of the second jaw 20 directed towards each other. Accordingly, the first jaw 10 and the second jaw 20 may be joined or connected to each other in a manner such that the engagement surface 16 of the first jaw 10 and the engagement surface 26 of the second jaw 20 may be facing each other. Further, the first jaw 10 and the second j aw 20 may be joined or connected to each other in a manner such that the first jaw 10 and the second jaw 20 are moveable relative to each other so as to move the engagement surface 16 of the first jaw 10 and the engagement surface 26 of the second jaw 20 towards each other to close the first jaw 10 and the second jaw 20, and to move the engagement surface 16 of the first jaw 10 and the engagement surface 26 of the second jaw 20 away from each other to open the first jaw 10 and the second jaw 20. According to various embodiments, the joint or the connection between the first jaw 10 and the second jaw 20 may be a revolute joint or a prismatic joint or any other suitable joints that may open and close the first jaw 10 and the second jaw 20 relative to each other.

According to various embodiments, the suturing end-effector 1 further includes a suture needle assembly 3. The suture needle assembly 3 includes a hollow suture needle 30. According to various embodiments, the hollow suture needle 30 has a first pointed tip 32, a second pointed tip 34 and an inner channel 36 extending through the hollow suture needle 30 from the first pointed tip 32 to the second pointed tip 34. Accordingly, the hollow suture needle 30 may be in the form of a hollow tube body with a slant-cut at both ends to form the first pointed tip 32 and the second pointed tip 34. The inner channel 36 of the hollow suture needle 30 may be a passage or conduit or an elongate hollow space surround or encircled by the hollow suture needle 30 and running the whole length of the hollow suture needle 30 from the first pointed tip 32 to the second pointed tip 34. According to various embodiments, the hollow suture needle 30 may be curved or may be straight or may be angled.

According to various embodiments, as shown in FIG. 1A, in a stowed disposition of the suturing end-effector 1 when in the transportation mode, the hollow suture needle 30 is clamped flat between the engagement surface 16 of the first jaw 10 and the engagement surface 26 of the second jaw 20. Accordingly, the hollow suture needle 30 may be laid horizontal or lengthwise with respect to the first jaw 10 and the second jaw 20 such that the hollow suture needle 30 may be sandwiched by the engagement surface 16 of the first jaw 10 and the engagement surface 26 of the second jaw 20 with a longitudinal direction (or a direction extending in the length) of the hollow suture needle 30 at least substantially parallel to the engagement surface 16 of the first jaw 10 and the engagement surface 26 of the second jaw 20. According to various embodiments, with the hollow suture needle 30 lying in a horizontal or lengthwise orientation in between the first jaw 10 and the second jaw 20, a minimum distance apart between the engagement surface 16 of the first jaw 10 and the engagement surface 26 of the second jaw 20 in the stowed disposition may be equal to an outer diameter of hollow suture needle 30 in order to clamp the hollow suture needle 30 between the first jaw 10 and the second jaw 20.

According to various embodiments, a guide wire 76 runs through the through-hole 11 of the first jaw 10, the inner channel 36 of the hollow suture needle 30, and the through-hole 21 of the second jaw 20 in a manner such that opening the first jaw 10 and the second jaw 20 relative to each other and tensioning the guide wire 76 cause the guide wire 76 to align the hollow suture needle 30 with the first pointed tip 32 of the hollow suture needle 30 directed towards the through-hole 11 of the first jaw 10 and the second pointed tip 34 of the hollow suture needle 30 directed towards the through-hole 21 of the second jaw 20. Accordingly, with the first jaw 10 and the second jaw 20 moved away to open up from each other and with the guide wire 76 tensioned to become taut, the guide wire 76 may be pulled tight or stretched across from the through-hole 11 of the first jaw 10 to the through-hole 21 of the second jaw 20. The guide wire 76 may be pulled at either or both ends for tensioning or tightening. Since the guide wire 76 runs through the inner channel 36 of the hollow suture needle 30 between the through-hole 11 of the first jaw 10 and the through-hole 21 of the second jaw 20, the hollow suture needle 30 may be suspended between the through-hole 11 of the first jaw 10 and the through-hole 21 of the second jaw 20 to form a suspended disposition of the suturing end-effector 1 when the first jaw 10 and the second jaw 20 open relative to each other and the guide wire 76 becomes taut or tighten.

According to various embodiment, as shown in FIG. 1B, in the suspended disposition of the suturing end-effector 1, the hollow suture needle 30 may be aligned upright or vertical with respect to the first jaw 10 and the second jaw 20 in a manner such that the first pointed tip 32 of the hollow suture needle 30 may be pointed or aimed towards the through-hole 11 of the first jaw 10 and the second pointed tip 34 of the hollow suture needle 30 may be pointed or aimed towards the through-hole 21 of the second jaw 20 with the help of the guide wire 76, which extends from the through-hole 11 of the first jaw 10 and enters into the inner channel 36 of the hollow suture needle 30 via the first pointed tip 32 of the hollow suture needle 30 and which exits from the inner channel 36 of the hollow suture needle 30 via the second pointed tip 34 of the hollow suture needle 30 to extend to through-hole 21 of the second jaw 20.

According to various embodiments, as shown in FIG. 1C, from the suspended disposition of the suturing end-effector 1, the first jaw 10 and the second jaw 20 may be closed relative to each other so as to form an inserted disposition of the suturing end-effector 1. As shown, in the inserted disposition of the suturing end-effector 1, the first pointed tip 32 of the hollow suture needle 30 is inserted through the through-hole 11 of the first jaw 10 and the second pointed tip 34 of the hollow suture needle 30 is inserted through the through-hole 21 of the second jaw 20. According to various other embodiments, it is understood that the first pointed tip 32 of the hollow suture needle 30 may inserted into the through-hole 11 of the first jaw 10 without protruding from the through-hole 11 of the first jaw 10, and the second pointed tip 34 of the hollow suture needle 30 may be inserted into the through-hole 21 of the second jaw 20 without protruding from the through-hole 21 of the second jaw 20. According to various embodiments, the hollow suture needle 30 may include a stopper or a limiter configured to limit an extend of insertion of the hollow suture needle 30 into the respective through-hole 11 of the first jaw 10 and through-hole 21 of the second jaw 20. According to various embodiments, the hollow suture needle 30 may be configured to be fitted into the through-hole 11 of the first jaw 10 in only one orientation of the hollow suture needle 30 with respect to the through-hole 11 of the first jaw 10. According to various embodiments, the hollow suture needle 30 may be configured to be fitted into the through-hole 21 of the second jaw 20 in only one orientation of the hollow suture needle 30 with respect to the through-hole 21 of the second jaw 20. According to various embodiments, as the first jaw and the second jaw close towards each other to form the inserted disposition, the guide wire 76 may be tensioned or tighten so as to facilitate the insertion of the respective first pointed tip 32 and second pointed tip 34 of the hollow suture needle 30 into the respective through-hole 11 of the first jaw 10 and through-hole 21 of the second jaw 20. The guide wire 76 may be tensioned or tighten via pulling either or both ends of the guide wire 76.

According to various embodiments, as shown in FIG. 1D, the hollow suture needle 30 may be locked to one of the first jaw 10 or the second jaw 20 with the guide wire 76 withdrawn, and the first jaw 10 and the second jaw 20 may be opened relative to each other such that the suturing end-effector 1 may form into a deployed disposition ready for performing suturing in the suturing mode. In the deployed disposition, the hollow suture needle 30 may be stood up or erected from one of the first jaw 10 or the second jaw 20. According to various embodiments, the first jaw 10 may include a locking element 15 configured to engage with the hollow suture needle 30 to lock the hollow suture needle 30 to the first jaw 10. According to various embodiments, the second jaw 20 may include a locking element 25 configured to engage with the hollow suture needle 30 to lock the hollow suture needle 30 to the second jaw 20. As shown in FIG. 1D, for example, the hollow suture needle 30 may be stood up or erected from the first jaw 10 with the first pointed tip 32 of the hollow suture needle 30 inserted through the through-hole 11 of the first jaw 10 and the locking element 15 of the first jaw 10 engaging the hollow suture needle 30 in a manner so as to lock the hollow suture needle 30 to the first jaw 10. On the other hand, the second pointed tip 34 of the hollow suture needle 30 may be free from the through-hole 21 of the second jaw 20 with the first jaw 10 and the second jaw 20 open relative to each other, the locking element 25 of the second jaw 20 withdraw from the hollow suture needle 30, and the guide wire 76 withdrawn completely from the suturing end-effector 1. According to various embodiments, the reverse may be achieved by reversing the engagement and withdrawal of the locking element 15 of the first jaw 10 and the locking element 25 of the second jaw 20 when the suturing end-effector 1 is in the inserted disposition of FIG. 1C.

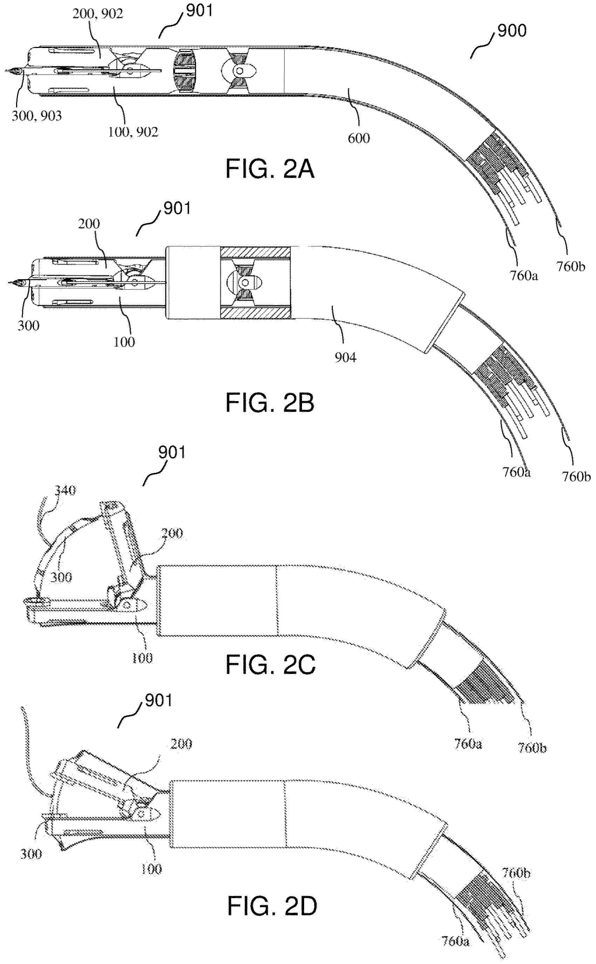

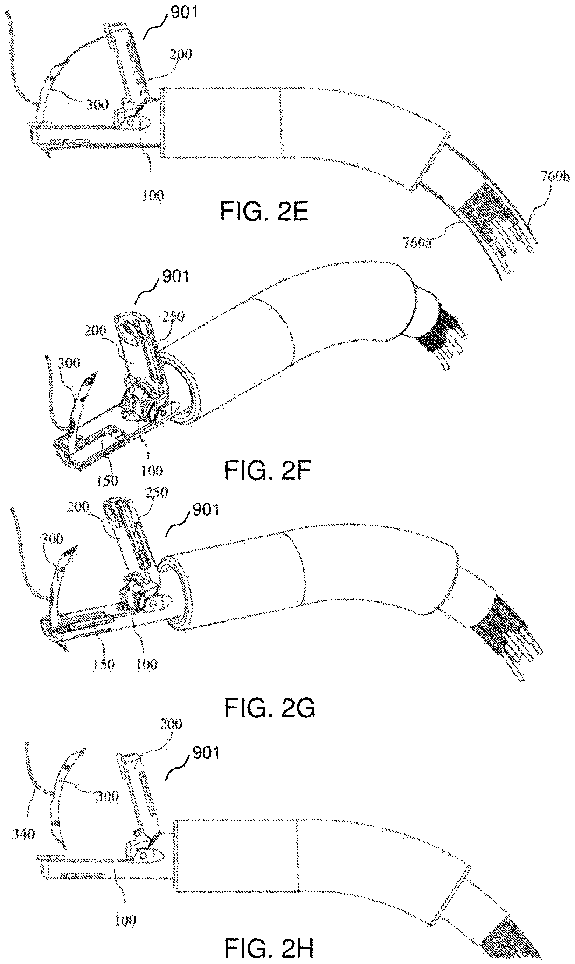

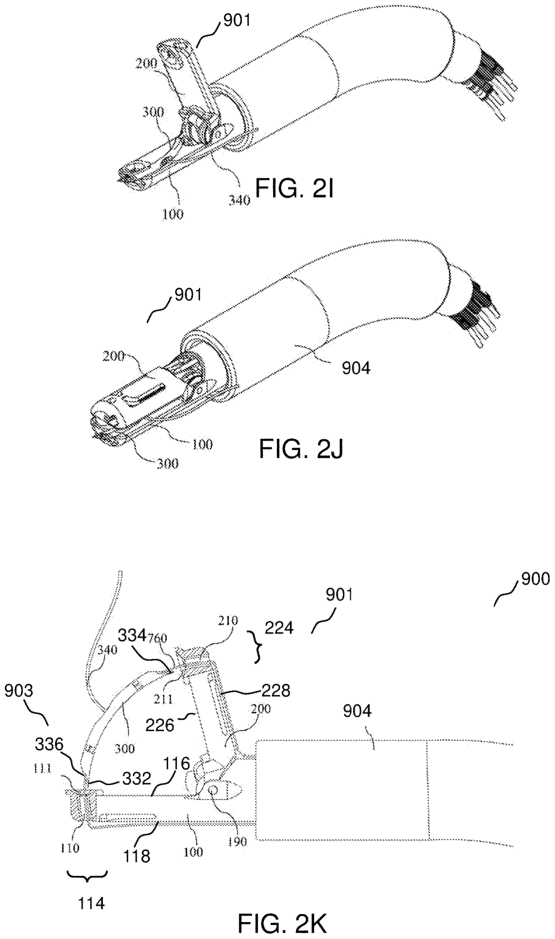

FIG. 2A to FIG. 2K show a suturing device 900 having a suturing end-effector 901 according to various embodiments. FIG. 2A to FIG. 2F show the suturing end-effector 901 of the suturing device 900 transforming or converting from the transportation mode to the suturing mode according to various embodiments. FIG. 2G to FIG. 2J show the suturing end-effector 901 of the suturing device 900 transforming or converting from the suturing mode back to the transportation mode according to various embodiments. Accordingly, switching between the suturing mode and transportation mode of the suturing device 900 is illustrated and described with reference to FIG. 2A to FIG. 2J. FIG. 2K shows a partial cross-sectional side view of FIG. 2C according to various embodiments.

According to various embodiments, the suturing end-effector 901 includes a jaw assembly 902. The jaw assembly 902 includes a first jaw 100 (or a lower jaw) and a second jaw 200 (or an upper jaw). Each of the first jaw 100 and the second jaw 200 may have an elongate body. According to various embodiments, the first jaw 100 includes a through-hole 110 disposed at an end portion 114 of the first jaw 100 (for example, see FIG. 2K). The end portion 114 of the first jaw 100 may be a free-end portion of the first jaw 100 distal to a joint or a connection 190 between the first jaw 100 and the second jaw 200. According to various embodiments, the through-hole 110 of the first jaw 100 extends through the first jaw 100 from an engagement surface 116 of the first jaw 100 to an opposite surface 118 of the first jaw 100. Accordingly, the through-hole 110 of the first jaw 100 may be a hole that goes all the way through the first jaw 100 from the engagement surface 116 of the first jaw 100 to the opposite surface 118 of the first jaw 100. The engagement surface 116 and the opposite surface 118 may be two surfaces on opposite sides of the first jaw 100. According to various embodiments, the second jaw 200 includes a through-hole 210 disposed at an end portion 224 of the second jaw 200 (for example, see FIG. 2K). The end portion 224 of the second jaw 200 may be a free-end portion of the second jaw 200 distal to the joint or the connection 190 between the first jaw 100 and the second jaw 200. According to various embodiments, the through-hole 210 of the second jaw 200 extends through the second jaw 200 from an engagement surface 226 of the second jaw 200 to an opposite surface 228 of the second jaw 200. Accordingly, the through-hole 210 of the second jaw 20 may be a hole that goes all the way through the second jaw 200 from the engagement surface 226 of the second jaw 200 to the opposite surface 228 of the second jaw 200. The engagement surface 226 and the opposite surface 228 may be two surfaces on opposite sides of the second jaw 200.

According to various embodiments, the first jaw 100 and the second jaw 200 are openable and closeable relative to each other with the engagement surface 116 of the first jaw 100 and the engagement surface 226 of the second jaw 200 directed towards each other. Accordingly, the first jaw 100 and the second jaw 200 may be joined or connected to each other in a manner such that the engagement surface 116 of the first jaw 100 and the engagement surface 226 of the second jaw 200 may be facing each other. Further, the first jaw 100 and the second jaw 200 may be joined or connected to each other in a manner such that the first jaw 100 and the second jaw 200 are moveable relative to each other so as to move the engagement surface 116 of the first jaw 100 and the engagement surface 226 of the second jaw 200 towards each other to close the first jaw 100 and the second jaw 200, and to move the engagement surface 116 of the first jaw 100 and the engagement surface 226 of the second jaw 200 away from each other to open the first jaw 100 and the second jaw 200. According to various embodiments, the joint or the connection 190 between the first jaw 100 and the second jaw 200 may be a revolute j oint or a pivot j oint such that the first jaw 100 and the second jaw 200 may open and close relative to each other via a pivoting motion or a rotating motion.

According to various embodiments, the suturing end-effector 901 further includes a suture needle assembly 903. The suture needle assembly 903 includes a hollow suture needle 300. According to various embodiments, the hollow suture needle 300 has a first pointed tip 332, a second pointed tip 334 and an inner channel 336 extending through the hollow suture needle 300 from the first pointed tip 332 to the second pointed tip 334. Accordingly, the hollow suture needle 300 may be in the form of a hollow tube body with a slant-cut at both ends to form the first pointed tip 332 and the second pointed tip 334. The inner channel 336 of the hollow suture needle 300 may be a passage or conduit or an elongate hollow space surround or encircled by the hollow suture needle 300 and running the whole length of the hollow suture needle 300 from the first pointed tip 332 to the second pointed tip 334. According to various embodiments, the hollow suture needle 300 may be curved.

According to various embodiments, in a stowed disposition of the suturing end-effector 901 when in the transportation mode, the hollow suture needle 300 is clamped flat between the engagement surface 116 of the first jaw 100 and the engagement surface 226 of the second jaw 200 as shown in FIG. 2A. Accordingly, the hollow suture needle 300 may be laid horizontal or lengthwise with respect to the first jaw 100 and the second jaw 200 such that the hollow suture needle 300 may be sandwiched by the engagement surface 116 of the first jaw 100 and the engagement surface 226 of the second jaw 200 with a longitudinal direction (or a direction extending in the length) of the hollow suture needle 300 at least substantially parallel to the engagement surface 116 of the first jaw 100 and the engagement surface 226 of the second jaw 200. According to various embodiments, with the hollow suture needle 300 lying in a horizontal or lengthwise orientation in between the first jaw 100 and the second jaw 200, a minimum distance apart between the engagement surface 116 of the first jaw 100 and the engagement surface 226 of the second jaw 200 in the stowed disposition may be equal to an outer diameter of hollow suture needle 300 in order to clamp the hollow suture needle 300 between the first jaw 100 and the second jaw 200.

As shown in FIG. 2A, according to various embodiments, the two jaws 100 and 200 may be completely closed, and the hollow suture needle 300 may not be locked to any jaw 100, 200 but may lay in-between the two closed jaws 100, 200 (the gap between the two closed jaws 100, 200 may be closed to the outer diameter of hollow suture needle 300). According to various embodiments, a guide wire 760 (e.g. a thin wire) may go through the hollow suture needle 300 and the two needle holes (in other words the through-hole 110 of the first jaw 100 and the through-hole 210 of the second jaw 200) on the jaws 100, 200. The two ends 760a, 760b of the guide wire 760 may be extended to the proximal side of the suturing device 900 and may be pulled by motors or hands. According to various embodiments, the guide wire 760 may stay outside or inside of a flexible shaft body 600 of the suturing device 900. According to various embodiments, two sheaths may also be used to protect the guide wire 760. According to various embodiments, the guide wire 760 may be stainless steel wire rope or nitinol wire which is flexible, strong, and less prone to kinking. According to various embodiments, with the hollow suture needle 300 lying between the two jaws 100, 200, the suturing device 900 may be small enough to be delivered through a tool channel of an endoscope 904 as shown in FIG. 2B. As shown in FIG. 2B, a tube is employed to denote the endoscope 904 for simplicity.

According to various embodiments, after reaching the target surgical site, the jaws 100, 200 may be opened, and the hollow suture needle 300 may be deployed to stand in-between the jaws 100, 200. Once tension is applied to the guide wire 760 by pulling its two sides (or ends) 760a and 760b, the suture needle 300 may be pointing to and guided to the needle hole (in other words the through-hole 110 of the first jaw 100 and the through-hole 210 of the second jaw 200) on either jaw 100, 200 as shown in FIG. 2C and FIG. 2K.

According to various embodiments, the guide wire 760 runs through the through-hole 110 of the first jaw 100, the inner channel 336 of the hollow suture needle 300, and the through-hole 221 of the second jaw 200 in a manner such that opening the first jaw 100 and the second jaw 200 relative to each other and tensioning the guide wire 760 cause the guide wire 760 to align the hollow suture needle 300 with the first pointed tip 332 of the hollow suture needle 300 directed towards the through-hole 110 of the first jaw 100 and the second pointed tip 334 of the hollow suture needle 300 directed towards the through-hole 221 of the second jaw 200. Accordingly, with the first jaw 100 and the second jaw 200 moved away to open up from each other and with the guide wire 760 tensioned to become taut, the guide wire 760 may be pulled tight or stretched across from the through-hole 110 of the first jaw 100 to the through-hole 210 of the second jaw 200. The guide wire 760 may be pulled at either or both ends 760a, 760b for tensioning or tightening. Since the guide wire 760 runs through the inner channel 336 of the hollow suture needle 300 between the through-hole 110 of the first jaw 100 and the through-hole 210 of the second jaw 20, the hollow suture needle 300 may be suspended between the through-hole 110 of the first jaw 100 and the through-hole 210 of the second jaw 200 to form a suspended disposition of the suturing end-effector 901 of the suturing device 900 when the first jaw 100 and the second jaw 200 open relative to each other and the guide wire 760 becomes taut or tighten. According to various embodiment, as shown in FIG. 2C and FIG. 2K, in the suspended disposition of the suturing end-effector 901 of the suturing device 900, the hollow suture needle 300 may be aligned upright or vertical with respect to the first jaw 100 and the second jaw 200 in a manner such that the first pointed tip 332 of the hollow suture needle 300 may be pointed or aimed towards the through-hole 110 of the first jaw 100 and the second pointed tip 334 of the hollow suture needle 300 may be pointed or aimed towards the through-hole 210 of the second jaw 200 with the help of the guide wire 760, which extends from the through-hole 110 of the first jaw 100 and enters into the inner channel 336 of the hollow suture needle 300 via the first pointed tip 332 of the hollow suture needle 300 and which exits from the inner channel 336 of the hollow suture needle 300 via the second pointed tip 334 of the hollow suture needle 300 to extend to through-hole 210 of the second jaw 200.

According to various embodiments, from the suspended disposition of the suturing end-effector 901 of the suturing device 900 as shown in FIG. 2C and FIG. 2K, the two jaws 100, 200 may be closed so that the hollow suture needle 300 is ready to be locked to one of the two jaws 100, 200 as shown in FIG. 2D. By moving a locking blade 150, 250 (see FIG. 2F and FIG. 2G) in either jaw 100, 200, the hollow suture needle 300 may be locked accordingly.

According to various embodiments, as shown in FIG. 2C and FIG. 2K, from the suspended disposition of the suturing end-effector 901 of the suturing device 900, the first jaw 100 and the second jaw 200 may be closed relative to each other so as to form an inserted disposition of the suturing end-effector 901 of the suturing device 900. As shown, in the inserted disposition of the suturing end-effector 901, the first pointed tip 332 of the hollow suture needle 300 may be inserted into or through the through-hole 110 of the first jaw 100 and the second pointed tip 334 of the hollow suture needle 300 may be inserted into or through the through-hole 210 of the second jaw 200. According to various embodiments, the hollow suture needle 300 may include a stopper or a limiter configured to limit an extend of insertion of the hollow suture needle 300 into the respective through-hole 110 of the first jaw 100 and through-hole 210 of the second jaw 200. According to various embodiments, the hollow suture needle 300 may be configured to be fitted into the through-hole 110 of the first jaw 100 in only one orientation of the hollow suture needle 300 with respect to the through-hole 110 of the first jaw 100. According to various embodiments, the hollow suture needle 300 may be configured to be fitted into the through-hole 210 of the second jaw 200 in only one orientation of the hollow suture needle 300 with respect to the through-hole 210 of the second jaw 200. According to various embodiments, as the first jaw 100 and the second jaw 200 close towards each other to form the inserted disposition, the guide wire 760 may be tensioned or tighten so as to facilitate the insertion of the respective first pointed tip 332 and second pointed tip 334 of the hollow suture needle 300 into the respective through-hole 110 of the first jaw 100 and through-hole 210 of the second jaw 200. The guide wire 760 may be tensioned or tighten via pulling either or both ends 760a, 760b of the guide wire 760.

According to various embodiments, to guide the hollow suture needle 300 into the respective needle holes (in other words the through-hole 110 of the first jaw 100 and the through-hole 210 of the second jaw 200), another surgical grasper beside the suturing device 900 may be used to grasp a suture thread 340 which is coupled to the hollow suture needle 300. By stretching the suture thread 340 distally along the longitudinal axis of the suturing device 900, the hollow suture needle 300 may be easily fitted into the desired needle hole.

According to various embodiments, the suture thread 340 may be coupled to the hollow suture needle 300. According to various embodiments, the suture thread 340 may be coupled to a mid-point of the hollow suture needle 300. According to various embodiments, the hollow suture needle 300 may be curved and the suture thread 340 may be coupled to the mid-point on the convex side of the curved hollow suture needle 300. According to various embodiments, the hollow suture needle 300, the through-hole 110 of the first jaw 100 and the through-hole 210 of the second jaw 200 may be configured such that the orientation of the hollow suture needle 300 for fitting into the respective through-holes 110, 210 of the first jaw 100 and the second jaw 200 is with the convex side of the curved hollow suture needle 300 directed away from the joint or the connection 190 between the first jaw 100 and the second jaw 200. Hence, by stretching the suture thread 340 distally away from the joint or the connection 190 between the first jaw 100 and the second jaw 200, the hollow suture needle 300 may be facilitated to be oriented in a manner so as to fit the first pointed tip 332 into the through-hole 110 of the first jaw 100 and to fit the second pointed tip 334 into the through-hole 210 of the second jaw 200.

As shown in FIG. 2E, according to various embodiments, the two jaws 100, 200 may be opened to confirm or check whether the hollow suture needle 300 is securely locked as desired. If the hollow suture needle 300 comes out from the desired receiving jaw 100, 200, the two jaws 100, 200 may be closed again and the hollow suture needle 300 may be locked again. Once hollow suture needle 300 is confirmed or verified to be locked, the guide wire 760 may be withdrawn by pulling one of its two ends, either 760a or 760b.

FIG. 2F shows the positions of the locking blades 150, 250 (or locking elements) in jaws 100, 200. As shown, the locking blade 150 may be at the distal end of the first jaw 100, locking the hollow suture needle to the first jaw 100. Meanwhile, the locking blade 250 may be at the proximal end of the second jaw 200, and it is not engaged with the hollow suture needle 300 (the mechanism for the movement of the locking blades will be described later). Till this stage, the suturing device 900 has been successfully transferred or transformed or converted from the transportation mode to the suturing mode, and it is ready for suturing tasks.

According to various embodiments, as shown in FIG. 2F, the hollow suture needle 300 may be locked to one of the first jaw 100 or the second jaw 200 with the guide wire 760 withdrawn, and the first jaw 100 and the second jaw 200 may be opened relative to each other such that the suturing end-effector 901 of the suturing device 900 may form into a deployed disposition ready for performing suturing in the suturing mode. In the deployed disposition, the hollow suture needle 300 may be stood up or erected from one of the first jaw 100 or the second jaw 200. According to various embodiments, the first jaw 100 may include the locking blade 150 (or locking element) configured to engage with the hollow suture needle 300 to lock the hollow suture needle 300 to the first jaw 100. According to various embodiments, the second jaw 200 may include a locking blade 250 (or locking element) configured to engage with the hollow suture needle 300 to lock the hollow suture needle 300 to the second jaw 200. As shown in FIG. 2F, for example, the hollow suture needle 300 may be stood up or erected from the first jaw 100 with the first pointed tip 332 of the hollow suture needle 300 inserted through the through-hole 110 of the first jaw 100 and the locking blade 150 of the first jaw 100 engaging the hollow suture needle 300 in a manner so as to lock the hollow suture needle 300 to the first jaw 100. On the other hand, the second pointed tip 334 of the hollow suture needle 300 may be free from the through-hole 210 of the second jaw 200 with the first jaw 100 and the second jaw 200 open relative to each other, the locking element 250 of the second jaw 200 withdraw from the hollow suture needle 300, and the guide wire 760 withdrawn completely from the suturing end-effector 901. According to various embodiments, the reverse may be achieved by reversing the engagement and withdrawal of the locking blade 150 of the first jaw 100 and the locking blade 250 of the second jaw 200 when the suturing end-effector 901 is in the inserted disposition of FIG. 2D.

Once the suturing task is done or completed, the suturing device 900 may be switched back to transportation mode so that it may be small enough to be withdrawn from the endoscope channel. To do that, for example, the locking blade 150 of the first jaw 100 may be moved back to disengage from the hollow suture needle 300, as shown in FIG. 2G. According to various embodiments, the locking blades 150, 250 of the first jaw 100 and the second jaw 200 may be withdrawn to disengage the hollow suture needle 300 from the first jaw 100 and the second jaw 200. The two jaws 100, 200 may then be opened, and the hollow suture needle 300 may thus be taken out by another surgical grasper besides the suturing device 900 (see FIG. 2H). According to various embodiments, the surgical grasper may either grab the suture thread 340 or the body of the hollow suture needle 300 to take the hollow suture needle 300 out from the respective jaw 100, 200. Next, the hollow suture needle 300 may be placed onto the respective engagement surfaces 116, 226 of the first jaw 100 or the second jaw 200, with the whole body of the hollow suture needle 300 lying on that jaw (for example, see FIG. 2I). Then, the two jaws 100, 200 may be closed to grab or clamp the hollow suture needle 300 securely. Till this stage, the suturing device 900 is switched from the suturing mode back to the transportation mode, and it is ready to be withdrawn from the tool channel of the endoscope 904 (see FIG. 2J).

Referring to FIG. 2K, according to various embodiments, the guide wire 760 may go through the needle hole on upper jaw (or the through-hole 210 of the second jaw 200), the inner channel 336 of the hollow suture needle 300, and then go through the needle hole on lower jaw (or the through-hole 110 of the first jaw 100). According to various embodiments, the hollow suture needle 300 and the two needle holes (i.e. the through-hole 110 of the first jaw 100 and the through-hole 210 of the second jaw 200) may have the same radius of curvature. According to various embodiments, the radius may be equal to a distance from a center of the needle hole to the axis of the joint or connection 190 (e.g. a shaft pin) between the first jaw 100 and the second jaw 200. According to various other embodiments, since the length of the needle holes (i.e. a length of the through-hole 110 of the first jaw 100 and a length of the through-hole 210 of the second jaw 200) are very short compared to their respective radius of curvature, thus their curved shapes (or curvature) may be approximated as straight. According to various embodiments, the through-hole 110 of the first jaw 100 may include a chamfer 111 about a rim of the through-hole 110 at the engagement surface 116 of the first jaw 100. According to various embodiments, the through-hole 210 of the second jaw 200 may include a chamfer 211 about a rim of the through-hole 210 at the engagement surface 226 of the second jaw 200. The respective chamfers 111 and 211 may help guide the needle tips (i.e. the first pointed tip 332 and the second pointed tip 334 of the hollow suture needle 300) into corresponding needle holes (i.e. the through-hole 110 of the first jaw 100 and the through-hole 210 of the second jaw 200).

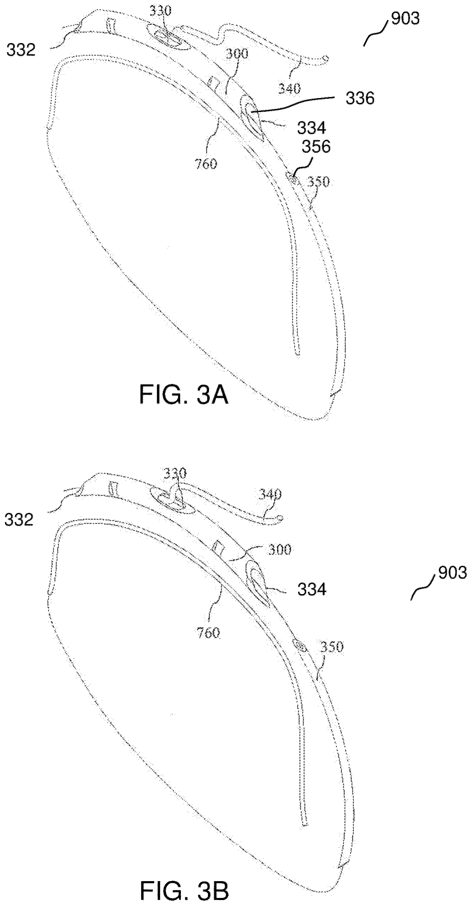

FIG. 3A to FIG. 3D shows the suture needle assembly 903 according to various embodiments. According to various embodiments, the suture needle assembly 903 may include the hollow suture needle 300 having the first pointed tip 332, the second pointed tip 334 and the inner channel 336 extending through the hollow suture needle 300 from the first pointed tip 332 to the second pointed tip 334. According to various embodiments, the hollow suture needle 300 may include an aperture 330 along the hollow suture needle 300 between the first pointed tip 332 and the second pointed tip 334. According to various embodiments, the aperture 330 may be at a mid-point of the hollow suture needle 300 between the first pointed tip 332 and the second pointed tip 334. According to various embodiments, the aperture 330 may be at the mid-point of the convex side of the hollow suture needle 300. According to various embodiments, the aperture 330 may provide access to the inner channel 336 of the hollow suture needle 300. Accordingly, the aperture 330 may provide an opening along the hollow suture needle 300 which opens into the inner channel 336 of the hollow suture needle 300.

According to various embodiments, the suture needle assembly 903 may further include the suture thread 340 coupled to the aperture 330 at the mid-point of the hollow suture needle 300. Accordingly, the suture thread 340 may be fixed or secured or attached or joined or coupled to the aperture 330 of the hollow suture needle 300 in a manner such that the suture thread 340 extends from the mid-point of the hollow suture needle 300. According to various embodiments, an end of the suture thread 340 may be inserted through the aperture 330 at the mid-point of the hollow suture needle 300 into the hollow suture needle 300 for coupling to the hollow suture needle 300. According to various embodiments, the suture needle assembly 903 may further include a tube 350. The tube 350 may be inserted through the inner channel 336 of the hollow suture needle 300 in a manner so as to wedge the inserted end of the suture thread 340 between an interior surface of the inner channel 336 of the hollow suture needle 300 and an exterior surface of the tube 350 so as to secure the suture thread 340 to the hollow suture needle 300. Accordingly, the suture thread 340 may be coupled to the aperture 330 at the mid-point of the hollow suture needle 300 via wedging or sandwiching or packing the inserted end of the suture thread 340 between the hollow suture needle 300 and the tube 350. According to various embodiments, the tube 350 may be hollow along the entire length with one opening on each end of the tube 350. According to various embodiments, the respective ends of the tube 350 may include a slant-cut such that the tube 350 may have pointed tips similar to that of the hollow suture needle 300. According to various embodiments, the tube 350 may be curved. According to various embodiments, the guide wire 760 may run through an inner channel 356 of the tube 350. Accordingly, the guide wire 760 may be inserted from one end of the tube 350 through the inner channel 356 of the tube 350 and to exit from the other end of the tube 350.

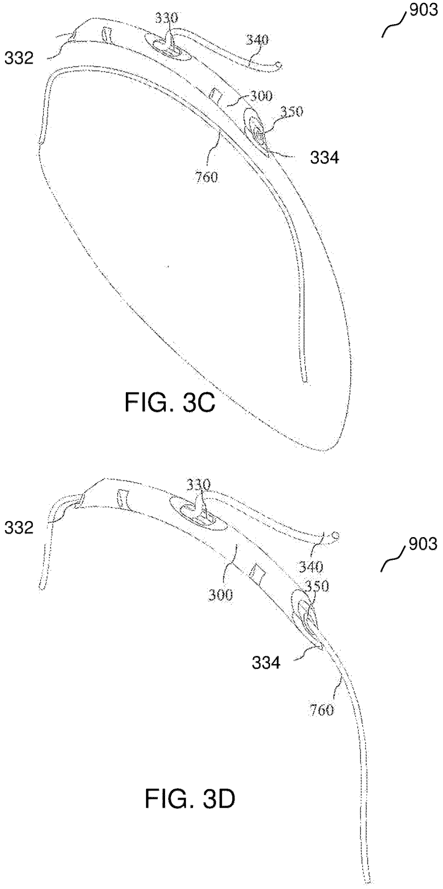

FIG. 3A to FIG. 3D shows the process of assembling the suture needle assembly 903 via integrating the hollow suture needle 300 with other components such as suture thread 340 and guide wire 760 for needle deployment to the suturing device 900. According to various embodiments, the hollow suture needle 300 may be made from a curved hollow tube, and there may be the aperture 330 (or a hole) in the middle wall of hollow suture needle 300 (for example, see FIG. 3A). As shown in FIG. 3A and FIG. 3B, according to various embodiments, the suture thread 340 may first be inserted into aperture 330. As shown in FIG. 3B and FIG. 3C, according to various embodiments, the thinner curved tube 350 may then be inserted into the inner channel 336 of the hollow suture needle 300 from the first pointed tip 332 to the second pointed tip 334. As shown in FIG. 3C and FIG. 3D, according to various embodiments, in the transportation mode of the suturing device 900, the guide wire 760 may be inserted throughout the inner channel 356 of tube 350 and thus the two ends of the guide wire 760 may be pulled to apply tension to the guide wire 760 for deployment of the hollow suture needle 300.

According to various embodiments, with the approach described above, both the tube 350 and the suture thread 340 may be cinched or squeezed together due to limited space inside the hollow suture needle 300, and the resulted friction may prevent them from sliding out of hollow suture needle 300. According to various embodiments, an outer diameter of tube 350 may be smaller than an inner diameter of hollow suture needle 300, and an inner diameter of the tube 350 may be larger than a diameter of the guide wire 760. According to various embodiments, to secure the tube 350 and the suture thread 340 inside the hollow suture needle 300 with more strength, adhesive glue may be applied to the outer surface of the tube 350, the suture thread 340, and/or the inner surface of the hollow suture needle 300. According to various embodiments, a radius of curvature of the tube 350 may be smaller than that of the hollow suture needle 300 such that the tube 350 may be significantly deformed after being inserted into the hollow suture needle 300 and thus a contact force between the hollow suture needle 300 and the tube 350 may be made larger. Accordingly, the increased contact force may thus increase the bonding strength among the hollow suture needle 300, the suture thread 340, and the tube 350.

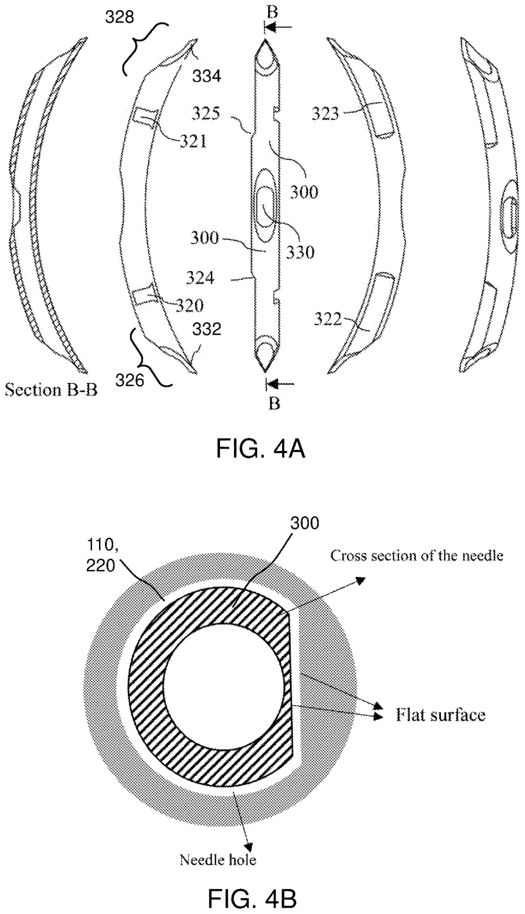

FIG. 4A shows a cross-sectional side view, a right side view, a front view, a left side view and a perspective front-left view of the hollow suture needle 300 according to various embodiments. According to various embodiments, the hollow suture needle 300 and the two needle holes (i.e. the through-hole 110 of the first jaw 100 and the through-hole 210 of the second jaw 200) may have the same radius of curvature, and the radius may be equal to the distance from the center of the needle hole to the joint or the connection 190 between the first jaw 100 and the second jaw 200 (also refer to FIG. 2K). According to various embodiments, the hollow suture needle 300 may be featured with two sharp tip points (i.e. the first pointed tip 332 and the second pointed tip 334). These two sharp tip points may ensure that the hollow suture needle 300 easily penetrate through tissue. According to various embodiments, there may be two recesses 320, 321(or slots) on the hollow suture needle 300. According to various embodiments, the recess 320 may be at a side of the first pointed tip 332, and the recess 321 may be at a side of the second pointed tip 334. According to various embodiments, the configuration, position, and size of the respective recesses 321, 320 may be configured to facilitate their engagement with their associated locking blades 150 and 250. Thus, according to various embodiments, the hollow suture needle 300 may include a first slot (or the first recess 320) across the hollow suture needle 300 and disposed towards the first pointed tip 332, and a second slot (or the second recess 321) across the hollow suture needle 300 and disposed towards the second pointed tip 334. According to various embodiments, each of the first slot and the second slot may be an elongate cut at least substantially perpendicular to the hollow suture needle 300. Further, each of the first slot and the second slot may extend from a convex side of the hollow suture needle 300 to a concave side of the hollow suture needle 300. Furthermore, each of the first slot and the second slot may be in the form of an indentation or groove or slit on the exterior surface of the hollow suture needle 300 without penetrating or puncturing into the inner channel 336 of the hollow suture needle 300. According to various embodiments, the respective locking blades 150, 250 of the first jaw 100 and the second jaw 200 may engage with the hollow suture needle 300 by intersecting with the respective first and second slots (the first recess 320 and the second recess 321) of the hollow suture needle 300 to lock the hollow suture needle 300 to the respective first and second jaws 100, 200. Accordingly, the respective locking blades 150, 250 of the first jaw 100 and the second jaw 200 may be inserted or slotted in to the respective first and second slots (the first recess 320 and the second recess 321) of the hollow suture needle 300 for locking the hollow suture needle 300.

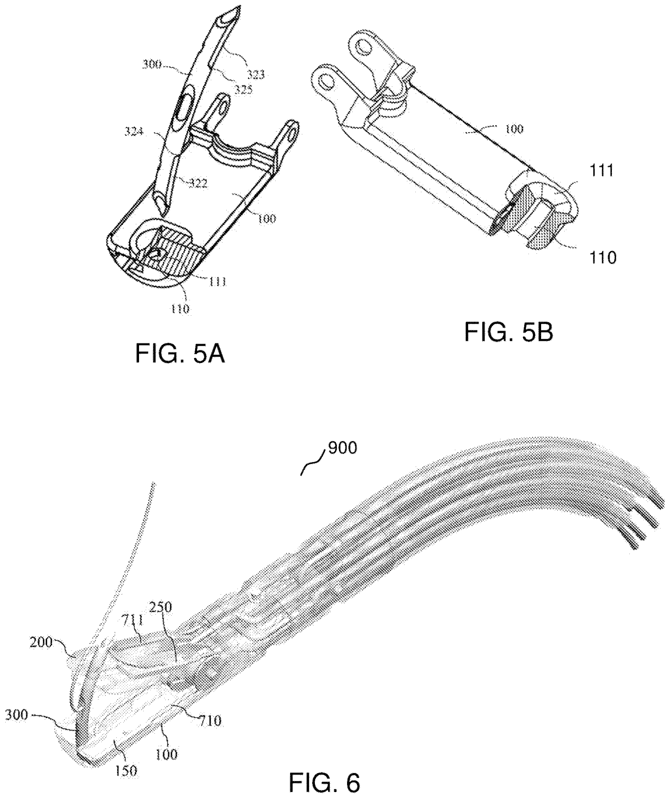

Referring back to FIG. 4A, according to various embodiments, on the other side of the hollow suture needle 300 (i.e. the side opposite to the side with the recesses 321 and 320), there may be two flat surfaces 322 and 323. According to various embodiments, the first surface 322 may be at the side of first pointed tip 332, and the second surface 323 may be at the side of second pointed tip 334. These two surfaces 322, 323 may ensure that the hollow suture needle 300 fits with the needle holes (i.e. the through-hole 110 of the first jaw 100 and the through-hole 210 of the second jaw 200) on the jaws 100, 200 and also prevent the hollow suture needle 300 from rotating undesirably. As can be seen from FIG. 4A, FIG. 4B and FIG. 5A, according to various embodiments, both the needle hole (i.e. the through-hole 110 of the first jaw 100 and the through-hole 210 of the second jaw 200) and the hollow suture needle 300 may have at least a flat section while the other portions are circular (or cylindrical). Due to these flat surfaces 322, 323, the hollow suture needle 300 may not be able to rotate in the respective needle hole (i.e. the through-hole 110 of the first jaw 100 or the through-hole 210 of the second jaw 200) of the respective jaw 100, 200 and thus may accurately point to the other needle hole on the opposite jaw 100, 200. According to various embodiments, details of the fitting between the hollow suture needle 300 and the needle holes (i.e. the through-hole 110 of the first jaw 100 and the through-hole 210 of the second jaw 200) may be seen from FIG. 5A and FIG. 5B. In addition, according to various embodiments, the respective surfaces 322, 323 may also include shoulders 324, 325, respectively, as can be seen from FIG. 4A and FIG. 4B. These shoulders 324, 325 may serve as stoppers which stop the hollow suture needle 300 at a desired depth when the hollow suture needle 300 is being inserted into the respective needle holes (i.e. the through-hole 110 of the first jaw 100 or the through-hole 210 of the second jaw 200). According to various embodiments, these two shoulders 324, 325, may stop the hollow suture needle 300 at a predetermined insertion depth such that the respective locking blades 150, 250 may slide into the respective recesses 320, 321 on the hollow suture needle 300 to lock the hollow suture needle 300 when the hollow suture needle 300 is inserted into respective first jaw 100 and the second jaw 200.

In other words, according to various embodiments, a cross-section outline of a first end section 326 of the hollow suture needle 300 towards the first pointed tip 336 may be of a shape without rotational symmetry, and a cross-section outline of the through-hole 110 of the first jaw 100 may be of a corresponding shape such that the first end section 326 of the hollow suture needle 300 fits into the through-hole 110 of the first jaw 100 in one orientation of the hollow suture needle 300 with respect to the through-hole 110 of the first jaw 100. Accordingly, there may be only one orientation of the hollow suture needle 300 with respect to the through-hole 110 of the first jaw 100 whereby the first end section 326 of the hollow suture needle 300 may fit into the through-hole 110 of the first jaw 100. Further, when the first end section 326 of the hollow suture needle 300 is fitted into the through-hole 110 of the first jaw 100, the hollow suture needle 300 may not be rotated with respect to the through-hole 110 of the first jaw 100. As shown in FIG. 4B, according to various embodiments, the cross-section outline of the first end section 326 of the hollow suture needle 300 may be of a shape resembling a circle with a cut away circular segment. Accordingly, the cross-section outline of the first end section 326 of the hollow suture needle 300 may be a partial circle with a straight portion and a rounded portion. As shown in FIG. 4A and FIG. 5A, according to various embodiments, the first end section 326 of the hollow suture needle 300 may include the flat surface 322 corresponding to the cut away circular segment of the cross-section outline of the first end section 326 of the hollow suture needle 300. According to various embodiments, the shoulder 324 may protrude from an edge 327 of the flat surface 322 distal to the first pointed tip 332 of the hollow suture needle 300.

Further, according to various embodiments, a cross-section outline of a second end section 328 of the hollow suture needle 300 towards the second pointed tip 334 may be of a shape without rotational symmetry, and a cross-section outline of the through-hole 210 of the second jaw 200 may be of a corresponding shape such that the second end section 328 of the hollow suture needle 300 fits into the through-hole of the second jaw in one orientation of the hollow suture needle with respect to the through-hole 210 of the second jaw 200. Accordingly, there may be only one orientation of the hollow suture needle 300 with respect to the through-hole 210 of the second jaw 200 whereby the second end section 328 of the hollow suture needle 300 may fit into the through-hole 210 of the second jaw 200. Further, when the second end section 328 of the hollow suture needle 300 is fitted into the through-hole 210 of the second jaw 200, the hollow suture needle 300 may not be rotated with respect to the through-hole 210 of the second jaw 200. According to various embodiments, similar to the first end section 326 of the hollow suture needle 300, the cross-section outline of the second end section 328 of the hollow suture needle 300 may be of a shape resembling a circle with a cut away circular segment. Accordingly, the cross-section outline of the second end section 328 of the hollow suture needle 300 may be a partial circle with a straight portion and a rounded portion. According to various embodiments, similar to the first end section 326 of the hollow suture needle 300, the second end section 328 of the hollow suture needle 300 may include the second flat surface 325 corresponding to the cut away circular segment of the cross-section outline of the second end section 328 of the hollow suture needle 300. According to various embodiments, the shoulder 325 may protrude from an edge 329 of the flat surface 323 distal to the second pointed tip 334 of the hollow suture needle 300.

FIG. 6 shows a transparent view of the suturing device 900 of FIG. 2A to FIG. 2K according to various embodiments. As shown, the hollow suture needle 300 may be locked on the first jaw 100 (or the lower jaw) by the locking blade 150 which may be advanced to the distal end of the first jaw 100 by a tendon 710. Meanwhile, the locking blade 250 may be withdrawn to the proximal end of the second jaw 200 (or the upper jaw) by the tendon 711 so that the locking blade 250 may not engage with the hollow suture needle 300. According to various embodiments, the tendon 710 and the tendon 711 may be the same tendon (i.e. the tendon 710 and the tendon 711 may be different portions of the same tendon). In FIG. 6, the portion of the tendon for the second jaw 200 (or the upper jaw) is numbered as 711 and the portion of the tendon for the first jaw 100 (or the lower jaw) is numbered as 710. In other words, the jaw assembly 902 of the suturing end-effector 901 of the suturing device 900 may include a tendon and pulley locking mechanism configured to alternately lock the hollow suture needle 300 to the first jaw 100 and the second jaw 200.

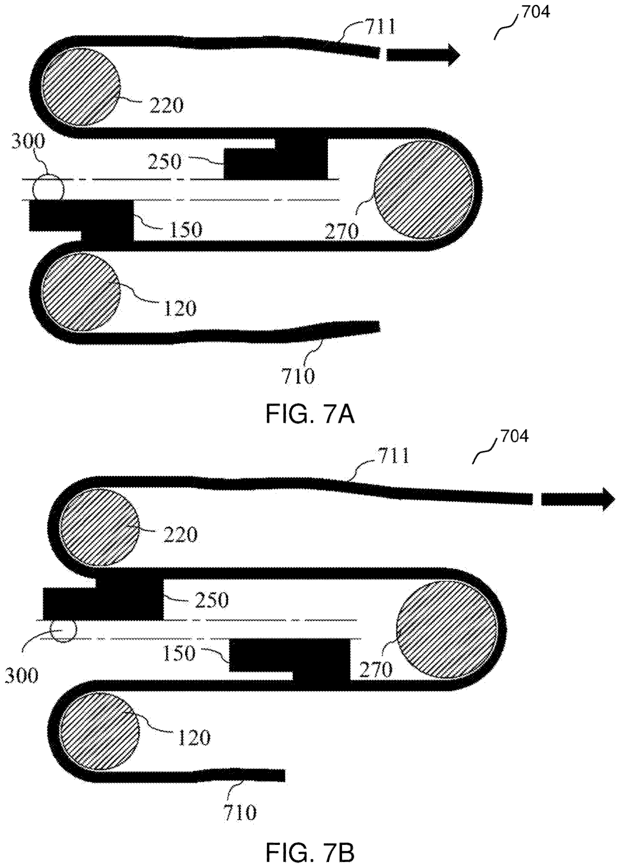

FIG. 7A and FIG. 7B show schematic diagram of a tendon and pulley locking mechanism 704 for the suturing device 900 according to various embodiments. While the tendon and pulley locking mechanism 704 is described herein in relation to the suturing device 900 having the hollow suture needle 300, it is understood that the tendon and pulley locking mechanism 704 may be applied in other suturing device which uses other types of suture needle that may not be hollow. According to various embodiments, there is also provided a suturing device including the tendon and pulley locking mechanism 704. Referring to FIG. 7A and FIG. 7B, the respective schematic diagram are showing how locking blades (or locking elements) in respective jaws of a suturing device may be moved by tendons according to various embodiments. As shown, the tendon 711 may extend from a proximal end of the suturing device to an upper jaw (or the second jaw) and wind around a pulley 220 on the upper jaw. According to various embodiments, the locking blade 250 may be fixed to the tendon 711 and may slide in a defined path which intersects with a portion of a cross-section of a suture needle when the suture needle is inserted into the upper jaw. The tendon 711 may then be wound around another pulley 270 which may be at a joint or a connection between the upper jaw and a lower jaw. The tendon may then go to a further pulley 120 on the lower jaw. Another locking blade 150 may also be fixed to the tendon. The locking blade 150 may also slide in a defined path which intersect with a portion of a cross-section of the suture needle when the suture needle is inserted into the lower jaw.

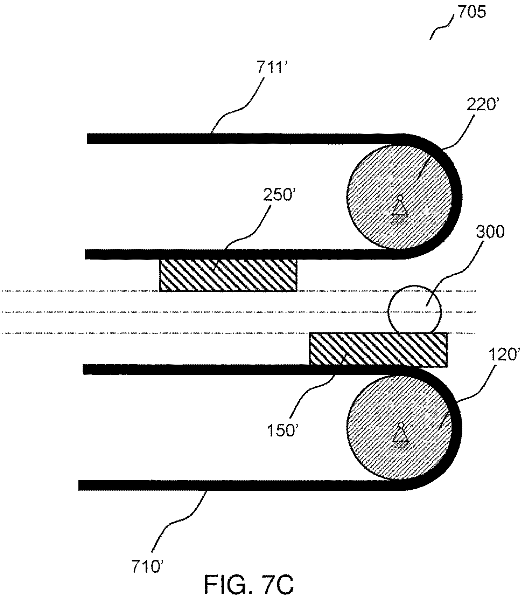

FIG. 7C show a schematic diagram of another tendon and pulley locking mechanism 705 for the suturing device 900 according to various embodiments. While the tendon and pulley locking mechanism 705 is described herein in relation to the suturing device 900 having the hollow suture needle 300, it is understood that the tendon and pulley locking mechanism 705 may be applied in other suturing device which uses other types of suture needle that may not be hollow. According to various embodiments, there is also provided a suturing device including the tendon and pulley locking mechanism 705. Referring to FIG. 7C, the schematic diagram is showing how locking blades (or locking elements) in respective jaws of a suturing device may be moved by two separate tendons 711', 710' according to various embodiments. As shown, the first tendon 711' may extend from a proximal end of the suturing device to an upper jaw (or the second jaw), wind around a pulley 220' on the upper jaw, and return to the proximal end of the suturing device. According to various embodiments, the locking blade 250' may be fixed to the first tendon 711' and may slide in a defined path which intersects with a portion of a cross-section of a suture needle when the suture needle is inserted into the upper jaw. Further, the second tendon 710' may extend from a proximal end of the suturing device to a lower jaw (or the first jaw), wind around a pulley 120' on the lower jaw and return to the proximal end of the suturing device. Another locking blade 150' may also be fixed to the second tendon 710'. The locking blade 150' may also slide in a defined path which intersect with a portion of a cross-section of the suture needle when the suture needle is inserted into the lower jaw. Accordingly, the blades 250', 150' may be independently controlled by the two separate tendons 710', 711'. In other words, the first tendon 711' may control the blade 250' in the upper jaw and the second tendon 710' may control the blade 150' in the lower jaw. Hence, the movements of the two blades 150', 250' may be separate and independent from each other.

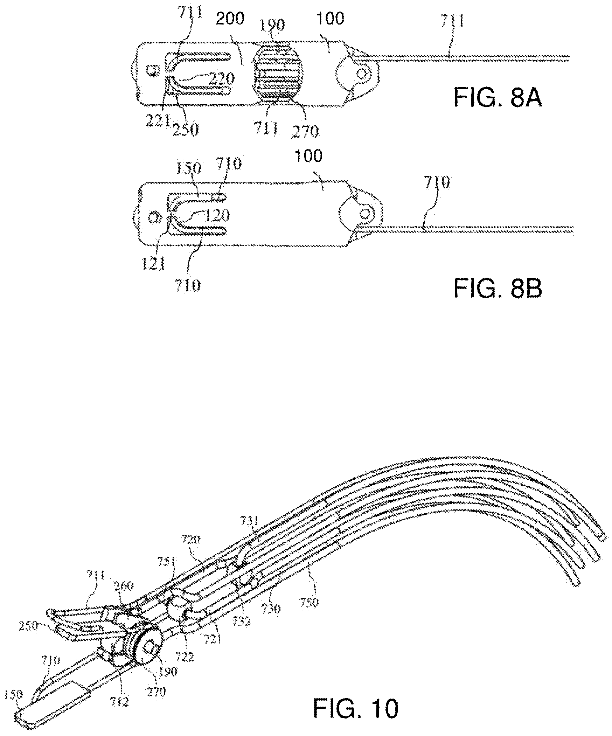

FIG. 8A and FIG. 8B show the tendon and pulley locking mechanism 704 of FIG. 7A and FIG. 7B applied in the suturing end-effector 901 according to various embodiments. FIG. 8A shows a top view of the second jaw 200 (or upper jaw) of the suturing end-effector 901 according to various embodiments. FIG. 8B shows a bottom view of the first jaw 100 (or lower jaw) of the suturing end-effector 901 according to various embodiments. In FIG. 8A, the locking blade 250 of the second jaw 200 may not be in engagement with the hollow suture needle 300 while the locking blade 150 of the first jaw 100 may be engaged with the hollow suture needle 300. According to various embodiments, by pulling tendon 711, the locking blade 250 may advance to engagement with the hollow suture needle 300 while the locking blade 150 may move away from the hollow suture needle 300 (as can be seen in FIG. 8B). In this way, the hollow suture needle 300 may be switched from one jaw to the other. According to various embodiments, pulling the tendon 710 may again engage the locking blade 150 of the first jaw 100 with the hollow suture needle 300 and may disengage the locking blade 250 of the second jaw 200 from the hollow suture needle 300. According to various embodiments, the pulleys 220, 270, 170 as described above may be pivotally rotatable or simply a smooth round contour which is not rotatable. Accordingly, the pulleys 220, 270, 170 may be either a rotating pulley or a fixed round contour.

In other words, according to various embodiments, the tendon and pulley locking mechanism 704 may include the first pulley 120 disposed in the first jaw 100, the second pulley 270 disposed at the joint or connection 190 between the first jaw 100 and the second jaw 200, and the third pulley 220 disposed in the second jaw 200. According to various embodiments, the tendon 710, 711 may be wound round the first pulley 120, the second pulley 270 and the third pulley 220 in a manner in which a first segment 710 of the tendon between the first pulley 120 and the second pulley 270 may be parallel to the engagement surface 116 of the first jaw 100 and a second segment 711 of the tendon between the second pulley 270 and the third pulley 220 may be parallel to the engagement surface 226 of the second jaw 200. According to various embodiments, the first locking blade 150 (or first locking element) may be attached to the first segment 710 of the tendon and the second locking blade 250 (or second locking element) may be attached to the second segment 711 of the tendon. According to various embodiments, pulling a first end of the tendon may cause the first locking blade 150 to move and lie across at least a part of the through-hole 110 of the first jaw 100 for engaging the hollow suture needle 300 to lock the hollow suture needle 300 to the first jaw 100 and may cause the second locking blade 250 to move away from the through-hole 210 of the second jaw 200 for disengaging the hollow suture needle 300. According to various embodiments, pulling a second end of the tendon may cause the second locking blade 250 to move and lie across at least a part of the through-hole 210 of the second jaw 200 for engaging the hollow suture needle 300 to lock the hollow suture needle 300 to the second jaw 200 and may cause the first locking blade 150 to move away from the through-hole 110 of the first jaw 100 for disengaging the hollow suture needle 300. According to various embodiments, each of the first locking blade 150 and the second locking blade 250 may be attached lengthwise to the respective segments 710, 711 of the tendon. Accordingly, each of the first locking blade 150 and the second locking blade 250 may be parallel to the respective segments 710, 711 of the tendon, which in turn is parallel to the respective engagement surfaces 116, 226 of the respective first and second jaws 100, 200.

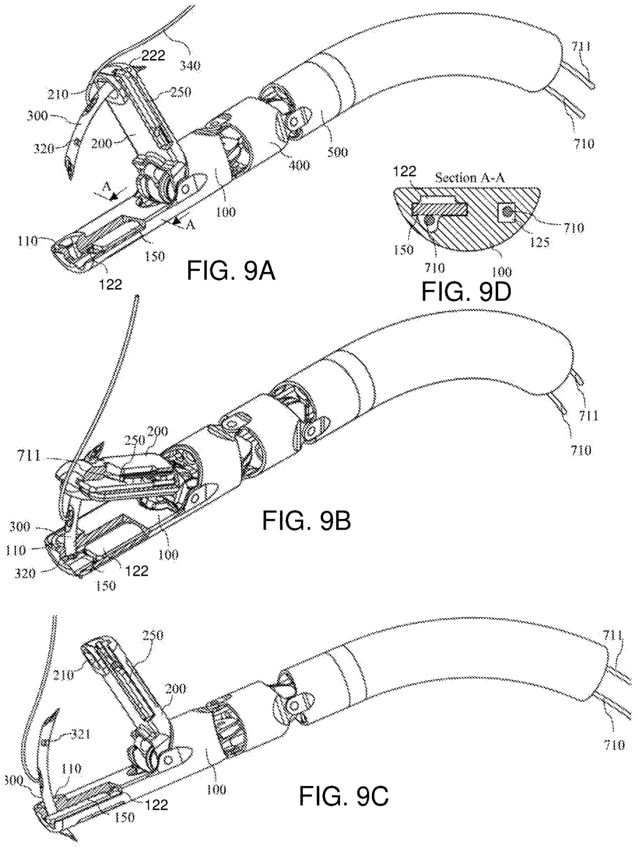

FIG. 9A to FIG. 9C show the locking blades 150, 250 movement for the switching of the hollow suture needle 300 between jaws 100, 200 according to various embodiments. As shown in FIG. 9A, the second jaw 200 (or the upper jaw) may be in the open position with respect to the first jaw 100 (or the lower jaw), and an upper end of the hollow suture needle 300 may be inserted into the needle hole (or the through-hole 210) on the second jaw 200. The locking blade 250, which may be slide along a channel 222 in the second jaw 200, may be at the distal end of the second jaw 200, locking the hollow suture needle 300 to the second jaw 200. Meanwhile, the locking blade 150, which may be slide along a channel 122 in the first jaw 100, may be at the proximal end of first jaw 100, allowing the hollow suture needle 300 to be inserted to the needle hole (or the through-hole 110) on the first jaw 100. When the second jaw 200 is rotated to the closed position, the hollow suture needle 300 moves along with the rotation of the second jaw 200, and the first pointed tip 332 of the hollow suture needle 300 goes into the needle hole (or the through-hole 110) on the first jaw 100 (see FIG. 9B). Subsequently, when the tendon 710 is pulled, and the locking blades 150, 250 fixed to the tendon start to move: the locking blade 150 may move forward to engage with the recess 320 (or the slot) on the hollow suture needle 300 while the locking blade 250 may be withdrawn back to disengage with the hollow suture needle 300 (See FIG. 9C). With the hollow suture needle 300 being locked to the first jaw 100 and unlocked from the second jaw 200, the second jaw 200 may thus be opened without bringing along the hollow suture needle 300. Similarly, the hollow suture needle 300 may be passed back to the second jaw 200 from the first jaw 100 by pulling the tendon 711 when the second jaw 200 is in the closed position. FIG. 9D is a cross-sectional view of the first jaw 100 with tendon 710 and the locking blade 150. As can be seen, according to various embodiments, the channel 122 may be configured so that both the tendon 710 and locking blade 150 may slide in the channel 122 with minimum friction force. Another channel 125 in the first jaw 100 may also be configured to allow tendon 710 to slide inside the first jaw 100. According to various embodiments, the second jaw 200 may also configured in the same way for the movement of the locking blade 250 and tendon 711.

Referring back to FIG. 8A, according to various embodiments, the tendon 711 may extends from the proximal end of the suturing device 900 through the second jaw 200 and wind around the pulley 220 (which may be a round contour) on the second jaw 200. According to various embodiments, the locking blade 250 may be crimped to the tendon 711 so that pulling the tendon 711 may move the locking blade 250 forward to the distal end of the second jaw 200. According to various embodiments, the tendon 711 may further extend from the locking blade 250 to wind around the other pulley 270 which may be rotatable on the joint 190 (such as a pin shaft). Then, the tendon 711 may be inserted into the first jaw 100, as shown in FIG. 8B. In FIG. 8B, the tendon is numbered as 710 to distinguish the portions on the first jaw 100 and the second jaw 200. According to various embodiments, the locking blade 150 may be fixed to the tendon 710 and wound around the further pulley 120 and eventually goes through the lower jaw 100 and back to the proximal end of the suturing device 900. According to various embodiments, rods 221 and 121 may prevent the tendon from dropping off the respective pulleys 220 and 120. According to various embodiments, the routing of tendons for the locking blades 150, 250 may be further detailed in FIG. 10 where the jaws 100, 200, joint links, and sheaths are removed to aid viewing the tendons.

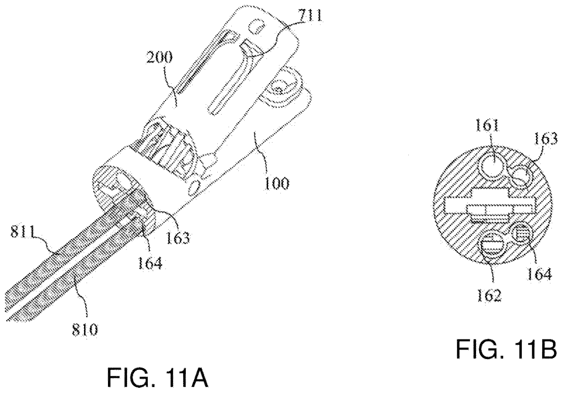

FIG. 11A shows a cut out view of a proximal end of the first jaw 100 (or the lower jaw) and FIG. 11B shows a cross-section view of the first jaw. According to various embodiments, there are four steps holes 161, 162, 163, 164 on the first jaw 100. According to various embodiments, each step hole 161, 162, 163, 164 may secure a corresponding sheath and may also allow the corresponding tendon to go through. For example, tendon 711 may go through step hole 163 to further extend to the second jaw 200, and sheath 811 may be secured by step hole 163. Further, the steps holes 161 and 162 may be for the tendon-sheath mechanism (e.g. tendons 750 and 751, sheaths 850 and 851) for moving the joint or connection 190 between the first jaw 100 and the second jaw 200.

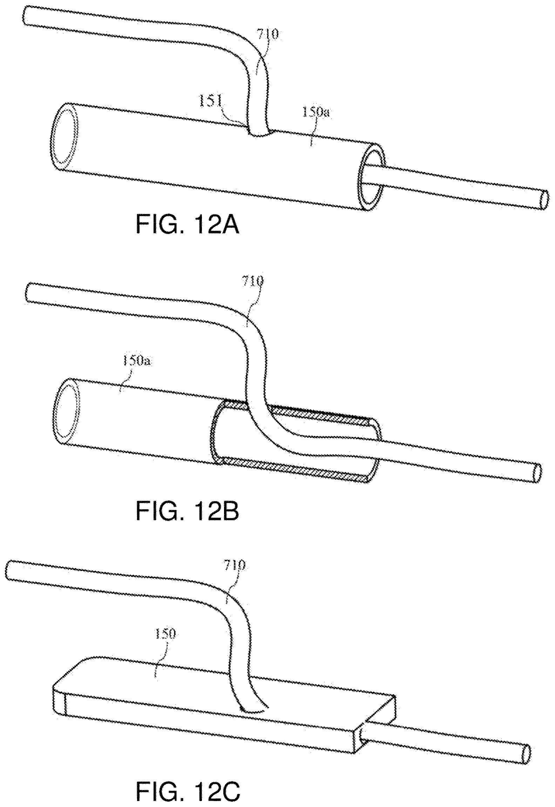

FIG. 12A to FIG. 12C show an approach to attaching the locking blade 150 to the tendon 710 according to various embodiments. According to various embodiments, the locking blade 150 may initially be a tube 150a having a hole 151. The tendon 710 may be inserted into a channel of tube 150a and then go through the hole 151, as can be seen from FIG. 12A and FIG. 12B. Subsequently, the tube 150a may be crimped to be flat together with the tendon 710. In this way, the tube 150a may be crimped into a thin locking blade 150 which may be tightly fixed to tendon 710 due to crimping. According to various embodiments, the locking blade 250 may also be attached to the tendon 711 in the same manner.

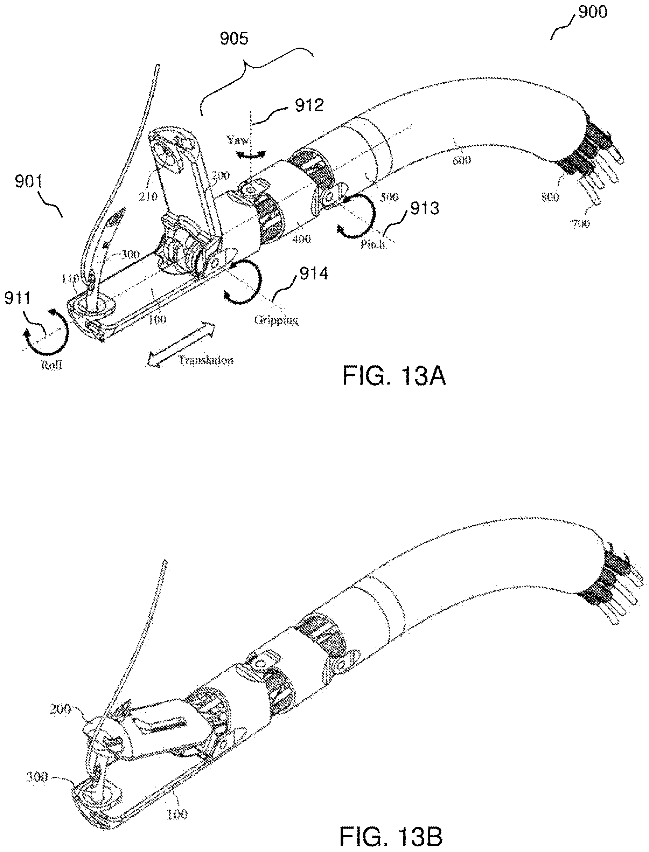

FIG. 13A and FIG. 13B shows the suturing device 900 according to various embodiments. According to various embodiments, the suturing device 900 may include a flexible tubular body shaft 600 which holds the tendons 700 and sheaths 800 inside and which connects with a neck assembly 905 of articulated joint links 400, 500. While the neck assembly 905 is described in relation to the suturing device 900 having the hollow suture needle 300, it is understood that the neck assembly 905 may be applied in other suturing device which uses other types of suture needle that may not be hollow. According to various embodiments, there is also provided a suturing device including the neck assembly 905. Referring back to FIG. 13A and FIG.13B, according to various embodiments, the joint links 400, 500 may be connected through pin joints and may provide the suturing device 900 with degrees of freedom in directions transverse to a longitudinal axis 911 of the suturing end-effector 901. According to various embodiments, at the distal end of the joint link 400, two jaws 100 and 200 may be pivotally connected, and a suture needle 300 may be operatively associated with the two jaws 100, 200 through the needle receiving recess/hole (i.e. the through-hole 110 of the first jaw 100 and the through-hole 210 of the second jaw 200) in each jaw 100, 200. According to various embodiments, the positions of the jaws 100, 200 and the joint links 400, 500 may be controllable by pulling the corresponding tendons 700 whose movement may either be controlled by motors or operators at the proximal side of the suturing device 900. According to various embodiments, depending on the applications requirement, the suturing device 900 may be configured to have multiple degrees of freedom: for example, translation along the longitudinal axis 911 of the suturing end-effector 901, gripping, yaw, pitch, roll (for applications where the required transmission system is short) as shown in FIG. 13A. According to various embodiments, the degrees of freedom may be modified by adding or removing joints, depending on the requirements of the applications.

Referring to FIG. 13B, the two jaws 100 and 200 may be in a closed position with the hollow suture needle 300 being inserted into the recess holes (i.e. the through-hole 110 of the first jaw 100 and the through-hole 210 of the second jaw 200) of both jaws 100, 200.

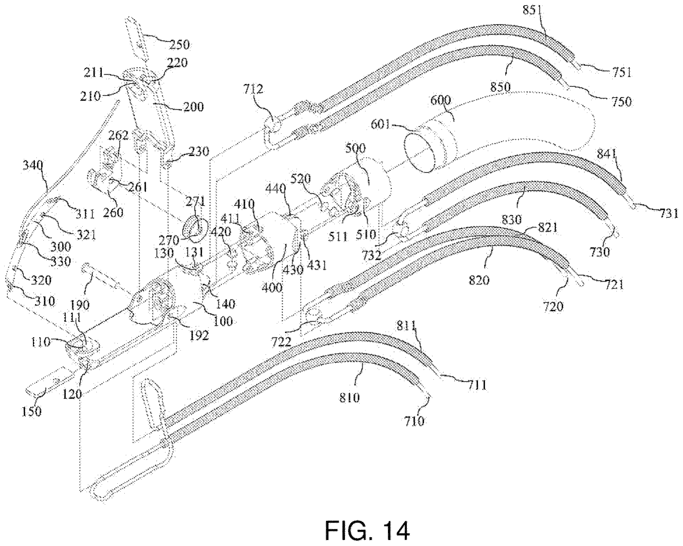

FIG. 14 shows an exploded view of the suturing device 900 according to various embodiments. According to various embodiments, each joint may be bi-directionally controlled through a pair of tendons. According to various embodiments, tendons 750, 751 routing around a pulley 260 that is assembled the joint or connection 190 between the second jaw 200 and the first jaw 100 may control the closing and opening of the second jaw 200 with respect to the first jaw 100. According to various embodiments, tendons 720, 721 routing around a pulley 140 between a first joint link 400 (e.g. a yaw link) and the two jaws 100, 200 may control the rotation of the two jaws 100, 200 with respect to a pair of pins 420 (or about a yaw axis 912 as shown in FIG. 13A). According to various embodiments, tendons 730 and 731 winding around a pulley 440 between the first joint link 400 (or the yaw link) and a second joint link 500 (or a pitch link) may control the rotation of the first joint link 400 with respect to a pair of pins 520 (or about a pitch axis 913 as shown in FIG. 13A). According to various embodiments, the tendons 720 and 721 may be two separated tendons or simply one tendon with two ends. Similarly, this may also apply to the tendons 710 and 711, 730 and 731, 750 and 751. According to various embodiments, for any joint link 400, 500, pulling one tendon may rotate the associated joint in one direction, and pulling the other opposite tendon may rotate the associated joint in the opposite direction. According to various embodiments, each pair of tendons may be a wire rope which may be inserted to one sheath, such as sheath 850, from the proximal end to the distal end. Subsequently, the same wire rope may be routed back to the proximal end through another sheath, e.g., sheath 851.

According to various embodiments, at the distal end, a crimping bead, e.g., 712, may be applied to the exposed tendon (720 and 721) between the two sheaths. According to various embodiments, the crimping bead may be supposed to be crimped tight enough onto the tendon to transform sufficient forces. According to various embodiments, the crimping bead 712 may be blocked by the pulley 260 so that the pulley 260 may rotate about the joint or connection 190 (which may be in the form of a pin shaft) by pulling either the tendon 751 or tendon 750. According to various embodiments, rotating the pulley 260 may result in the same rotation of the second jaw 200 because the pulley 260 may be fixed to the second jaw 200 through a pair of flaps 262 and may share the same rotation axis (for example axis 914 as shown in FIG. 13 A) with the second jaw 200 through the shaft pin of the joint or connection 190. According to various embodiments, in the same way, tendons 720 and 721 may actuate the first jaw through the pulley 140. According to various embodiments, in the same way, tendons 730 and 731 may actuate the j oint between the first j oint link 400 and the second joint link 500 through the pulley 440. According to various embodiments, the second jaw 200 may be connected to the first jaw 100 through the pin at the joint or connection 190. The pin may go through a pair of pin holes 192 on the first jaw 100, a shaft hole 261 on the pulley 260, a pair of joint holes 230 on the second jaw 200, and a hole 271 on the pulley 270.

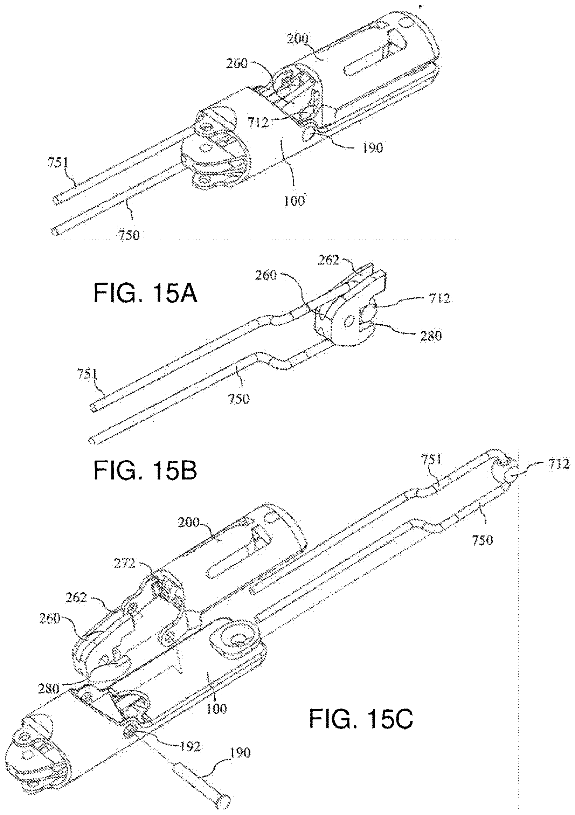

FIG. 15A to FIG. 15C depicts how the tendons 750 and 751 are connected to a grasper (or the jaw assembly 902) having the jaws 100, 200 according to various embodiments. According to various embodiments, the tendon 751 may extend through the hole 161 in the first jaw 100 (or the lower jaw) and then winds around the pulley 260 and come back through another hole 162 on the first jaw 100 as tendon 750 (refer to FIG. 11B for the holes 161, 162 on first jaw 100). According to various embodiments, the tendons 750, 751 may be crimped by a crimping bead 712 which may be positioned at a recess 280 on the pulley 260. According to various embodiments, the recess 280 may block the crimping bead 280 and thus the pulley 260 may rotate as the tendons are pulled (see FIG. 15B). According to various embodiments, on the pulley 260, there may be a pair of flaps 262 which may be inserted into the recess 272 on the second jaw 200. Accordingly, once the joint or connection 190 is assembled via the shaft pin, the pulley 260 and second jaw 200 may always rotate together (see FIG. 15C).

According to various embodiments, FIG. 15B shows an example of how a pair of tendons may be fixed to a pulley through a crimping bead. According to various embodiments, the tendons 750, 751 may be wound around the pulley 260 and may be crimped by the crimping bead 712 which may be blocked by the recess 280 on the pulley 260. Further, the two flaps 262 on the pulley 260 may also prevent the tendons 750, 751 from dropping off the pulley 260. According to various embodiments, other pulleys such as the pulley 140 for the joint between the first jaw 100 and the first joint link 400 as well as the pulley 440 for the joint between the first joint link 400 and the second joint link 500, respectively, may be connected to their associated tendons in the same way.

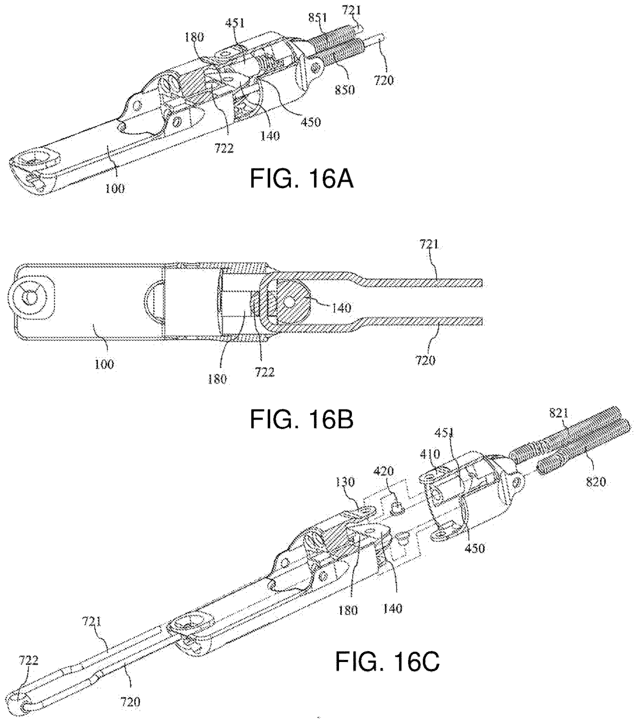

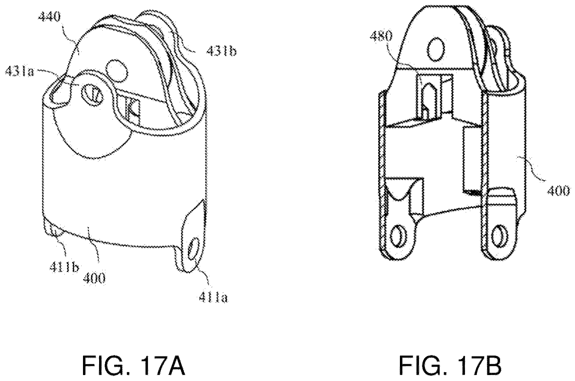

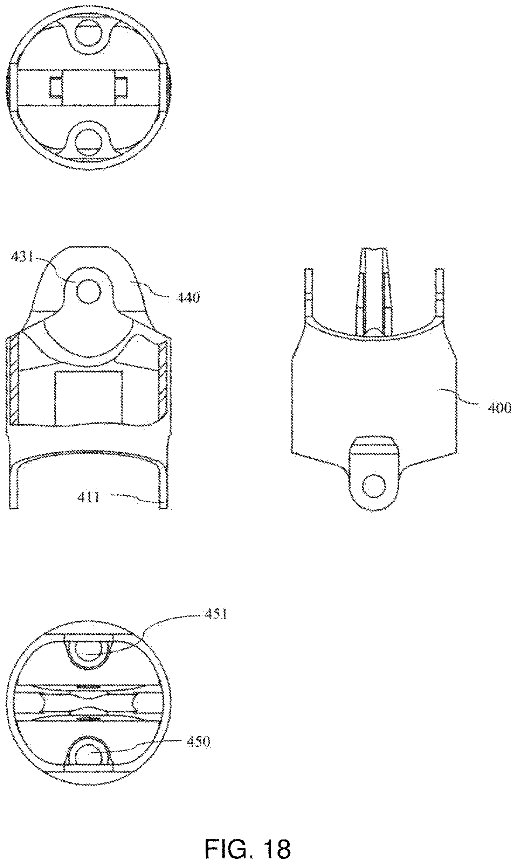

FIG. 16A to FIG. 16C show another pair of tendons 720, 721 may be employed to steer the first jaw 100 relative to the first joint link 400 according to various embodiments. According to various embodiments, on the first joint link 400, there may be two step hole structures 450, 451 through which the tendons 720, 721 may be inserted, respectively, and the sheaths 820, 821 may be fixed (see FIG. 16A and FIG. 16C). According to various embodiments, the tendons 720, 721 may be crimped by a crimping bead 722 which may be blocked by a recess 180 (see FIG. 16A and FIG. 16C) on the pulley 140. According to various embodiments, the first joint link 400 and first jaw 100 may be pivotally connected through a pair of pins 420 (see FIG. 16C). Accordingly, pulling either tendon 720 or tendon 721 may rotate the first jaw 100 (for example, about the yaw axis 912 in FIG. 13A) along with the jay assembly 902 with respect to the pins 420. FIG. 17A and FIG. 17B shows a perspective view and a broken-out section view of the first joint link 400 according to various embodiments. FIG. 18 shows a front view, a top view, a bottom view and a left side view of the first joint link 400 according to various embodiments.

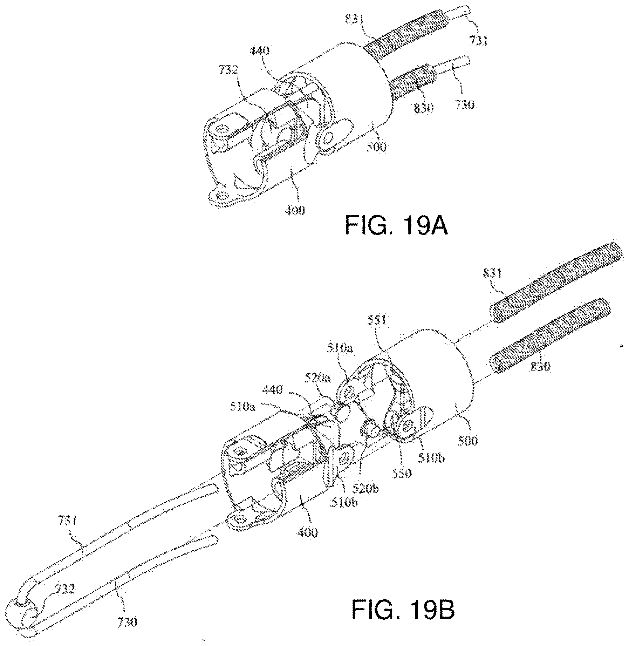

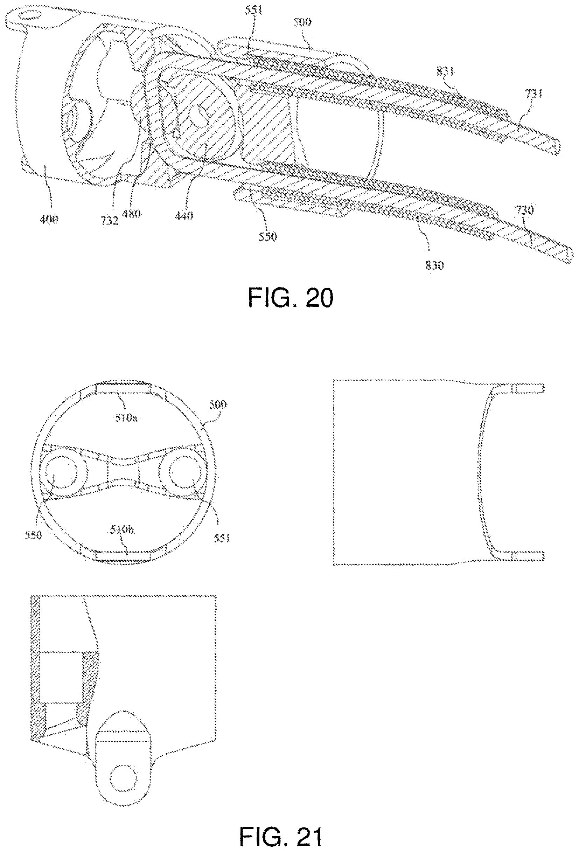

FIG. 19A and FIG. 19B show a further pair of tendons 730, 731 may be employed to steer the first joint link 400 relative to the second joint link 500 according to various embodiments. As shown in FIG. 19A and FIG. 19B, according to various embodiments, the second joint link 500 may further be pivotally connected to the proximal side of the first joint link 400 at joint flaps 510a, 510b by pins 520a, 520b respectively. According to various embodiments, the tendons 730, 731 may be inserted through step holes 550, 551 respectively, and may be crimped by a crimping bead 732 at a recess 480 (see FIG. 20) of the pulley 440. According to various embodiments, the first joint link 400 and the second joint link 500 may be pivotally connected by pins 520a, 520b (see FIG. 19B). According to various embodiments, pulling either tendon 730, 731 may rotate the first joint link 400 (for example about the pitch axis 913 in FIG. 13A) with respect to the pins 520a, 520b. FIG. 20 shows a broken-out section view of the second joint link 500 according to various embodiments. FIG. 21 shows a front view, a top view and a left view of the second joint link 500 according to various embodiments.

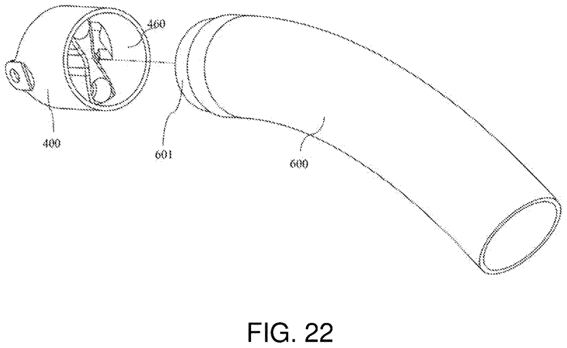

FIG. 22 shows a bonding between the shaft body 600 and the second joint link 500 according to various embodiments. As shown, according to various embodiments, a distal end 601 of the flexible shaft body 600, with a step shoulder, may be inserted into a proximal side of the second joint link 500. According to various embodiments, the bonding between the shaft body 600 and the second joint link 500 may be secured with a tight fitting or welding.

Referring back to FIG. 13A to FIG. 22, in other words, according to various embodiments, the suturing device 900 may include the jaw assembly 902 having the first jaw 100 and the second jaw 200 connected to each other via a pivoting j oint (for example, the joint or connection 190 which may be formed by the shaft pin) in a manner so as to provide a pivoting motion relative to each other about an pivot axis (for example the pivot axis 914) of the pivoting joint such that the first jaw 100 and the second jaw 200 may be openable and closeable relative to each other. Further, according to various embodiments, the suturing device 900 may include a yaw link (for example the first joint link 400) coupled to the jaw assembly 902 via a yaw joint (for example, may be formed by the pair of pins 420) in a manner so as to provide a yaw motion to the jaw assembly 902 relative to the yaw link about a yaw axis (for example the yaw axis 912) of the jaw assembly 902. Furthermore, according to various embodiments, suturing device 900 may include a pitch link (for example the second joint link 500) coupled to the jaw assembly 902 via a pitch joint (for example, may be formed by the pins 520a, 520b) in a manner so as to provide a pitch motion to the jaw assembly 902 relative to the pitch link about a pitch axis (for example the pitch axis 913) of the jaw assembly. According to various embodiments, the pitch link may be coupled to the jaw assembly 902 through the yaw link. According to various other embodiments, the yaw link may be coupled to the jaw assembly 902 through the pitch link.

According to various embodiments, the pivoting joint of the jaw assembly 902 and/or the yaw joint of the yaw link and/or the pitch joint of the pitch link, each may include a tendon and pulley mechanism configured to control the respective motion (i.e. the pivot motion of the pivoting j oint, the yaw motion of the yaw j oint, and the pitch motion of the pitch joint). According to various embodiments, each of the tendon and pulley mechanism may include a pulley rotatably disposed coaxially with the respective axes and a tendon (or tendons) wound round the pulley in a manner such that pulling either sides of the tendon (or either tendons) with respect to the pulley may cause the pulley to rotate in respective direction so as to generate the respective motion of the respective joints.

According to various embodiments the pulley may include a retaining element and the tendon may include a node retained in the retaining element of the pulley in a manner such that the node engages the retaining element to rotate the pulley as the tendon is being pulled. According to various embodiments, the retaining element of the pulley may be a catch element or a holder element and the node of the tendon may be a swell or a bulge on the tendon. Accordingly, the catch element or the holder element may retain or hold the swell or the bulge on the tendon. According to various embodiments, the retaining element of the pulley may include a recessed portion cut into a sector of the pulley and the node of the tendon may include a crimping bead crimped onto the tendon.

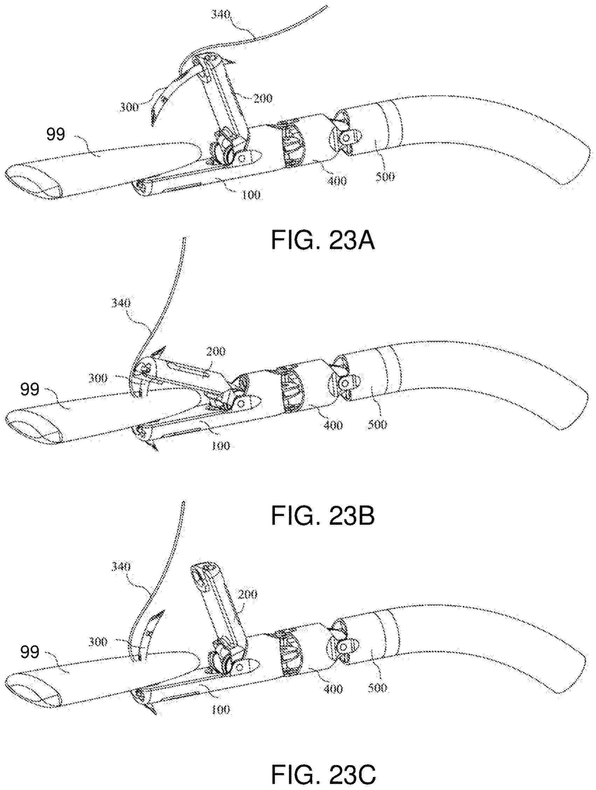

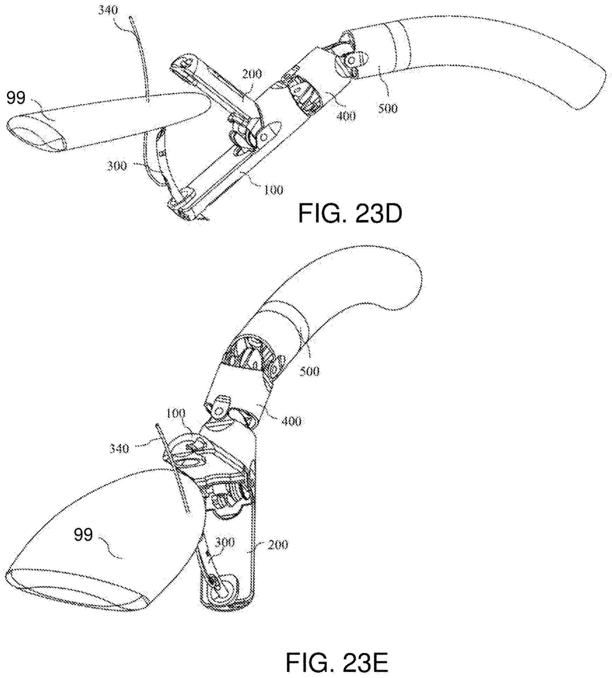

FIG. 23A to FIG. 23E show a suturing process using the suturing device 900 according to various embodiments. As shown in FIG. 23A, the two jaws 100, 200 may be open, and the upper end of hollow suture needle 300 with the suture thread 340 may be engaged with the second jaw 200 (or the upper jaw). The hollow suture needle 300 may be locked to the second jaw 200 by the locking blade 250 inside the second jaw 200 (for example, in the manner as described earlier with reference to the mechanism for blade movement). A piece of tissue 99 may be placed in between the hollow suture needle 300 and the first jaw 100 (or the lower jaw). As shown in FIG. 23B, by closing the second jaw 200, the hollow suture needle 300 with a sharp tip point (for example, the first pointed tip 332) may penetrate through the tissue 99, and that sharp end may also goes into the recess hole (for example, the through-hole 110) on the first jaw 100. Then, the hollow suture needle 300 may be unlocked from the second jaw 200 and meanwhile being locked to first jaw 100 so that the switching of hollow suture needle engagement may be achieved. Next, the second jaw 200 may be open again (see FIG. 23C) and the first joint link 400 may be activated to rotate relatively to the second joint link 500 (for example, about the pitch axis 913) so that the tissue 99 slides off the hollow suture needle 300 which drags the suture thread 340 through the tissue 99 (see FIG. 23D). According to various embodiments, the process as shown in FIG. 23A to FIG. 23D may be repetitively done to generate running stitches. According to various embodiments, the first jaw 100, together with the jaw assembly 902, may also be rotated with respect to the first joint link 400 (for example, about the yaw axis 912) so that another stitch may be made at another location of the tissue 99 (see FIG. 23E). According to various embodiments, in the suturing process, another collaborative grasper may be employed to facilitate manipulating the tissue 99. According to various embodiments, the entire suturing device 900 may also be pushed, pulled, twisted as a whole for tissue manipulation.

Various embodiments have provided a suturing end-effector or a suturing device having a suturing end-effector which may efficiently perform endoscopic closure for the GI tract, featuring with triangulation capability and fast change of tools during operations. Various embodiments have provided a suturing end-effector or a suturing device having a suturing end-effector with an outer diameter of the end-effector in transportation mode significantly smaller than that in the suturing mode. Thus, various embodiments in the transportation mode may be small enough to be delivered to the surgical site through the tool channel of the endoscope. Various embodiments have been provided with a more reliable force and motion transmission system for locking and unlocking of suture needle from the jaws of the suturing end-effector. Various embodiments have multiple actuated degrees of freedom, which may be actuated by flexible tendon and pulley mechanisms, such that the suturing end-effector or the suturing device does not rely on the movement of the endoscope to move from one site to the other, and does not rely on other tools for triangulation.

While the invention has been particularly shown and described with reference to specific embodiments, it should be understood by those skilled in the art that various changes, modification, variation in form and detail may be made therein without departing from the scope of the invention as defined by the appended claims. The scope of the invention is thus indicated by the appended claims.

## Claims

1. A suturing end-effector (1, 901), comprising:
a jaw assembly (2, 902) comprising
a first jaw (10, 100) having a through-hole (11, 110) which is disposed at an end portion (14, 114) of the first jaw (10, 100) and which extends through the first jaw (10, 100) from an engagement surface (16, 116) of the first jaw (10, 100) to an opposite surface (18, 118) of the first jaw (10, 100),
a second jaw (20, 200) having a through-hole (21, 210) which is disposed at an end portion (24, 224) of the second jaw (20, 200) and which extends through the second jaw (20, 200) from an engagement surface (26, 226) of the second jaw (20, 200) to an opposite surface (28, 228) of the second jaw (20, 200),
wherein the first jaw (10, 100) and the second jaw (20, 200) are openable and closeable relative to each other with the engagement surface (16, 116) of the first jaw (10, 100) and the engagement surface (26, 226) of the second jaw (20, 200) directed towards each other,
**characterized in that** the suturing end-effector (1, 901) further comprising
a suture needle assembly (3, 903) comprising a hollow suture needle (30, 300) having a first pointed tip (32, 332), a second pointed tip (34, 334) and an inner channel (36, 336) extending through the hollow suture needle (30, 300) from the first pointed tip (32, 332) to the second pointed tip (34, 334),
wherein, in a stowed disposition of the suturing end-effector (1, 901), the hollow suture needle (30, 300)_is clamped flat between the engagement surface (16, 116) of the first jaw (10, 100) and the engagement surface (26, 226) of the second jaw (20, 200), and a guide wire (76, 760) runs through the through-hole (11, 110) of the first jaw (10, 100), the inner channel (36, 336) of the hollow suture needle (30, 300), and the through-hole (21,210) of the second jaw (20, 200) in a manner such that opening the first jaw (10, 100) and the second jaw (20, 200) relative to each other and tensioning the guide wire (76, 760) causes the guide wire (76, 760) to align the hollow suture needle (30, 300) with the first pointed tip (32, 332) of the hollow suture needle (30, 300) directed towards the through-hole (11, 110) of the first jaw (10, 100) and the second pointed tip (34, 334) of the hollow suture needle (30, 300) directed towards the through-hole (21, 210) of the second jaw (20, 200).

2. The suturing end-effector (901) as claimed in claim 1, wherein the hollow suture needle (300) has an aperture (330) along the hollow suture needle (300) between the first pointed tip (332) and the second pointed tip (334), the aperture (330) providing access to the inner channel (336) of the hollow suture needle (300), and wherein the suture needle assembly (903) further comprises a suture thread (340) coupled to the aperture (330) along the hollow suture needle (300).

3. The suturing end-effector (901) as claimed in claim 2, wherein an end of the suture thread (340) is inserted through the aperture (330) along the hollow suture needle (300) into the hollow suture needle (300), and wherein the suture needle assembly (300) further comprises a tube (350) inserted through the inner channel (336) of the hollow suture needle (300) in a manner so as to wedge the inserted end of the suture thread (340) between an interior surface of the inner channel (336) of the hollow suture needle (300) and an exterior surface of the tube (350) so as to secure the suture thread (340) to the hollow suture needle (300), and wherein the guide wire (760) runs through an inner channel (356) of the tube (350).

4. The suturing end-effector (901) as claimed in any one of claims 1 to 3, wherein a cross-section outline of a first end section (326) of the hollow suture needle (300) towards the first pointed tip (332) is of a shape without rotational symmetry and a cross-section outline of the through-hole (110) of the first jaw (100) is of a corresponding shape such that the first end section (326) of the hollow suture needle (300) fits into the through-hole (110) of the first jaw (100) in one orientation of the hollow suture needle (300) with respect to the through-hole (110) of the first jaw (100), preferably, wherein the cross-section outline of the first end section (326) of the hollow suture needle (300) is of a shape resembling a circle with a cut away circular segment, and preferably, wherein the first end section (326) of the hollow suture needle (300) comprises a flat surface (322) corresponding to the cut away circular segment of the cross-section outline of the first end section (326) of the hollow suture needle (300), and wherein a shoulder (324) protrudes from an edge (327) of the flat surface (322) distal to the first pointed tip (332).

5. The suturing end-effector (901) as claimed in any one of claims 1 to 4, wherein a cross-section outline of a second end section (328) of the hollow suture needle (300) towards the second pointed tip (334) is of a shape without rotational symmetry and a cross-section outline of the through-hole (210) of the second jaw (200) is of a corresponding shape such that the second end section (328) of the hollow suture needle (300) fits into the through-hole (210) of the second jaw (200) in one orientation of the hollow suture needle (300) with respect to the through-hole (210) of the second jaw (200), preferably wherein the cross-section outline of the second end section (328)of the hollow suture needle (300) is of a shape resembling a circle with a cut away circular segment, and preferably, wherein the second end section (328) of the hollow suture needle (300) comprises a flat surface (323) corresponding to the cut away circular segment of the cross-section outline of the second end section (328) of the hollow suture needle (300), and wherein a shoulder (325) protrudes from an edge (329) of the flat surface (323) distal to the second pointed tip (300).

6. The suturing end-effector (901) as claimed in any one of claims 1 to 5, wherein the first jaw (100) and the second jaw (200) are connected to each other via a pivoting joint (190) in a manner so as to provide a pivoting motion relative to each other about an pivot axis (914) of the pivoting joint (190) such that the first jaw (100) and the second jaw (200) are openable and closeable relative to each other.

7. The suturing end-effector (901) as claimed in any one of claims 1 to 6, further comprising a yaw link (400) coupled to the jaw assembly (902) via a yaw joint (420) in a manner so as to provide a yaw motion to the jaw assembly (902) relative to the yaw link (400) about a yaw axis (912) of the jaw assembly (902), and/or a pitch link (500) coupled to the jaw assembly (902) via a pitch joint (520a, 502b) in a manner so as to provide a pitch motion to the jaw assembly (902) relative to the pitch link about a pitch axis (913) of the jaw assembly (902).

8. The suturing end-effector (901) as claimed claim 6 or 7, wherein the pivoting joint (190) and/or the yaw joint (420) and/or the pitch joint (520), each comprises a tendon and pulley mechanism (704) configured to control the respective motion, wherein the tendon and pulley mechanism (704) comprises a pulley (260, 140, 440) rotatably disposed coaxially with the respective axes (914, 912, 913) and a tendon (750, 751, 730, 731, 720, 721) wound round the pulley (260, 140, 440) in a manner such that pulling either sides of the tendon (750, 751, 730, 731, 720, 721) with respect to the pulley (260, 140, 440) causes the pulley (260, 140, 440) to rotate in respective direction so as to generate the respective motion of the respective joints, preferably, wherein the pulley (260, 140, 440) comprises a retaining element and the tendon (750, 751, 730, 731, 720, 721) comprises a node retained in the retaining element of the pulley (260, 140, 440) in a manner such that the node engages the retaining element to rotate the pulley (260, 140, 440) as the tendon (750, 751, 730, 731, 720, 721) is being pulled, and preferably, wherein the retaining element of the pulley (260, 140, 440) comprises a recessed portion cut into a sector of the pulley (260, 140, 440) and the node of the tendon (750, 751, 730, 731, 720, 721) comprises a crimping bead (712, 722, 732) crimped onto the tendon (750, 751, 730, 731, 720, 721).

9. The suturing end-effector (901) as claimed in any one of claims 1 to 8, wherein the jaw assembly (902) further comprises a tendon and pulley locking mechanism (704) configured to alternately lock the hollow suture needle (300) to the first jaw (100) and the second jaw (200),
wherein the tendon and pulley locking mechanism (704) comprises a first pulley (120) disposed in the first jaw (100), a second pulley (270) disposed at a joint (190) between the first jaw (100) and the second jaw (200), and a third pulley (220) disposed in the second jaw (200),
wherein a tendon (710, 711) is wound round the first pulley (120), the second pulley (270) and the third pulley (220) in a manner in which a first segment (710) of the tendon between the first pulley (120) and the second pulley (270) is parallel to the engagement surface (116) of the first jaw (100) and a second segment (711) of the tendon between the second pulley (270) and the third pulley (220) is parallel to the engagement surface (226) of the second jaw (200),
wherein a first locking element (150) is attached to the first segment (710) of the tendon and a second locking element (250) is attached to the second segment (711) of the tendon,
wherein pulling a first end of the tendon causes the first locking element (150) to move and lie across at least a part of the through-hole (110) of the first jaw (100) for engaging the hollow suture needle (300) to lock the hollow suture needle (300) to the first jaw (100) and causes the second locking element (250) to move away from the through-hole (210) of the second jaw (200) for disengaging the hollow suture needle (300), and
wherein pulling a second end of the tendon causes the second locking element (250) to move and lie across at least a part of the through-hole (210) of the second jaw (200) for engaging the hollow suture needle (300) to lock the hollow suture needle (300) to the second jaw (200) and causes the first locking element (150) to move away from the through-hole (110) of the first jaw (100) for disengaging the hollow suture needle (300).

10. The suturing end-effector as claimed in in any one of claims 1 to 8, wherein the jaw assembly further comprises a tendon and pulley locking mechanism 705 configured to alternately lock the hollow suture needle (300) to the first jaw (100) and the second jaw (200),
wherein the tendon and pulley locking mechanism (705) comprises
a first locking sub-mechanism comprising a first pulley (120') disposed in the first jaw (100) and a first tendon (710') wound round the first pulley (120'),
a second locking sub-mechanism comprising a second pulley (220') disposed in the second jaw (200) and a second tendon (711') wound round the second pulley (220'),
wherein a first locking element (150') is attached to the first tendon (710') and a second locking element (250') is attached to the second tendon (711'),
wherein pulling a first end of the first tendon (710') causes the first locking element (150') to move and lie across at least a part of the through-hole (110) of the first jaw (100) for engaging the hollow suture needle (300) to lock the hollow suture needle (300 to the first jaw (100) and pulling a second end of the first tendon (710') causes the first locking element (150') to move away from the through-hole (110) of the first jaw (100) for disengaging the hollow suture needle (300),
wherein pulling a first end of the second tendon (711') causes the second locking element (250') to move and lie across at least a part of the through-hole (210) of the second jaw (200) for engaging the hollow suture needle (300) to lock the hollow suture needle (300) to the second jaw (200) and pulling a second end of the second tendon (711') causes the second locking element (250') to move away from the through-hole (2100 of the second jaw (200) for disengaging the hollow suture needle (300),
wherein the first locking sub-mechanism and the second locking sub-mechanism are independently operable.

11. The suturing end-effector (901) as claimed in claim 9 or 10,
wherein the first locking element (150) and the second locking element (250), each comprises a locking blade attached lengthwise to respective segment of the respective tendon, and
wherein the hollow suture needle (300) comprises a first slot (320) across the hollow suture needle (300) and disposed towards the first pointed tip (332), and a second slot (321) across the hollow suture needle (300) and disposed towards the second pointed tip (334),
wherein respective locking blades engages the hollow suture needle (300) by intersecting with the respective first and second slots (320,321) of the hollow suture needle (300) to lock the hollow suture needle (300) to the respective first and second jaws (100, 200).

12. The suturing end-effector (901) as claimed in any one of claims 9 to 11, wherein each pulley (120, 270, 220, 120', 220') of the tendon and pulley locking mechanism (704, 705) comprises a rotating pulley or a fixed round contour.

## Patentansprüche

1. Nähen-Endeffektor (1, 901), aufweisend:
eine Backeneinrichtung (2, 902), welche aufweist:
eine erste Backe (10, 100), welche ein Durchgangsloch (11, 110) hat, welches an einem Endabschnitt (14, 114) der ersten Backe (10, 100) angeordnet ist und welches sich durch die erste Backe (10, 100) hindurch erstreckt von einer Eingriffsfläche (16, 116) der ersten Backe (10, 100) aus hin zu einer entgegengesetzten Fläche (18, 118) der ersten Backe (10, 100),
eine zweite Backe (20, 200), welche ein Durchgangsloch (21, 210) hat, welches an einem Endabschnitt (24, 224) der zweiten Backe (20, 200) angeordnet ist und welches sich durch die zweite Backe (20, 200) hindurch erstreckt von einer Eingriffsfläche (26, 226) der zweiten Backe (20, 200) aus hin zu einer entgegengesetzten Fläche (28, 228) der zweiten Backe (20, 200),
wobei die erste Backe (10, 100) und die zweite Backe (20, 200) relativ zueinander öffenbar und schließbar sind, wobei die Eingriffsfläche (16, 116) der ersten Backe (10, 100) und die Eingriffsfläche (26, 226) der zweiten Backe (20, 200) in Richtung hin zu einander gerichtet sind,
**dadurch gekennzeichnet, dass** der Nähen-Endeffektor (1, 901) ferner aufweist:
eine Nähnadeleinrichtung (3, 903), welche eine hohle Nähnadel (30, 300) aufweist, welche eine erste spitze Spitze (32, 332), eine zweite spitze Spitze (34, 334) und einen Innenkanal (36, 336) hat, welcher sich durch die hohle Nähnadel (30, 300) hindurch von der ersten spitzen Spitze (32, 332) aus hin zu der zweiten spitzen Spitze (34, 334) erstreckt,
wobei, in einer verstauten Anordnung des Vernähen-Endeffektors (1, 901), die hohle Nähnadel (30, 300) flach zwischen der Eingriffsfläche (16, 116) der ersten Backe (10, 100) und der Eingriffsfläche (26, 226) der zweiten Backe (20, 200) geklemmt ist, und wobei ein Führungsdraht (76, 760) durch das Durchgangsloch (11, 110) der ersten Backe (10, 100), den Innenkanal (36, 336) der hohlen Nähnadel (30, 300) und das Durchgangsloch (21, 210) der zweiten Backe (20, 200) in einer Weise derart hindurchgeht, dass ein Öffnen der ersten Backe (10, 100) und der zweiten Backe (20, 200) relativ zueinander und ein Spannen des Führungsdrahts (76, 760) bewirkt, dass der Führungsdraht (76, 760) die hohle Nähnadel (30, 300) ausrichtet, wobei die erste spitze Spitze (32, 332) der hohlen Nähnadel (30, 300) in Richtung hin zu dem Durchgangsloch (11, 110) der ersten Backe (10, 100) gerichtet ist und die zweite spitze Spitze (34, 334) der Nähnadel (30, 300) in Richtung hin zu dem Durchgangsloch (21, 210) der zweiten Backe (20, 200) gerichtet ist.

2. Nähen-Endeffektor (901) gemäß Anspruch 1, wobei die hohle Nähnadel (300) eine Öffnung (330) entlang von der hohlen Nähnadel (300) zwischen der ersten spitzen Spitze (332) und der zweiten spitzen Spitze (334) hat, wobei die Öffnung (330) einen Zugang zu dem Innenkanal (336) der hohlen Nähnadel (300) bereitstellt, und wobei die Nähnadeleinrichtung (903) ferner einen Nähfaden (340) aufweist, welcher mit der Öffnung (330) entlang von der hohlen Nähnadel (300) gekoppelt ist.

3. Nähen-Endeffektor (901) gemäß Anspruch 2, wobei ein Ende von dem Nähfaden (340) durch die Öffnung (330) hindurch entlang von der hohlen Nähnadel (300) in die hohle Nähnadel (300) hinein eingeführt ist, und wobei die Nähnadeleinrichtung (300) ferner einen Schlauch (350) aufweist, welcher durch den Innenkanal (336) der hohlen Nähnadel (300) hindurch in einer Weise eingeführt ist, um das eingeführte Ende von dem Nähfaden (340) zwischen einer Innenfläche des Innenkanals (336) der hohlen Nähnadel (300) und einer Außenfläche des Schlauches (350) festzuklemmen, um den Nähfaden (340) an der hohlen Nähnadel (300) zu sichern, und wobei der Führungsdraht (760) durch einen Innenkanal (356) des Schlauches (350) hindurchgeht.

4. Nähen-Endeffektor (901) gemäß einem von den Ansprüchen 1 bis 3, wobei ein Querschnittsumriss von einem ersten Endabschnitt (326) der hohlen Nähnadel (300) in Richtung hin zu der ersten spitzen Spitze (332) von einer Form ohne Rotationssymmetrie ist und ein Querschnittsumriss von dem Durchgangsloch der ersten Backe (100) von einer korrespondierenden Form ist derart, dass der erste Endabschnitt (326) der hohlen Nähnadel (300) in das Durchgangsloch (110) der ersten Backe (100) hineinpasst in einer Orientierung der hohlen Nähnadel (300) mit Bezug auf das Durchgangsloch (110) der ersten Backe (100), wobei vorzugsweise der Querschnittsumriss von dem ersten Endabschnitt (326) der hohlen Nähnadel (300) von einer Form ist, welche einem Kreis mit einem weggeschnittenen Kreissegment ähnelt, und wobei vorzugsweise der erste Endabschnitt (326) der hohlen Nähnadel (300) eine ebene Fläche (322) aufweist, welche mit dem weggeschnittenen Kreissegment des Querschnittsumrisses von dem ersten Endabschnitt (326) der hohlen Nähnadel (300) korrespondiert, und wobei eine Schulter (324) von einem Rand (327) der ebenen Fläche (322) aus distal hin zu der ersten spitzen Spitze (332) hervorsteht.

5. Nähen-Endeffektor (901) gemäß einem von den Ansprüchen 1 bis 4, wobei ein Querschnittsumriss von einem zweiten Endabschnitt (328) der hohlen Nähnadel (300) in Richtung hin zu der zweiten spitzen Spitze (334) von einer Form ohne Rotationssymmetrie ist und ein Querschnittsumriss von dem Durchgangsloch (210) der zweiten Backe (200) von einer korrespondierenden Form ist derart, dass der zweite Endabschnitt (328) der hohlen Nähnadel (300) in das Durchgangsloch (210) der zweiten Backe (200) hineinpasst in einer Orientierung der hohlen Nähnadel (300) mit Bezug auf das Durchgangsloch (210) der zweiten Backe (200), wobei vorzugsweise der Querschnittsumriss von dem zweiten Endabschnitt (328) der hohlen Nähnadel (300) von einer Form ist, welche einem Kreis mit einem weggeschnittenen Kreissegment ähnelt, und wobei vorzugsweise der zweite Endabschnitt (328) der hohlen Nähnadel (300) eine ebene Fläche (323) aufweist, welche mit dem weggeschnittenen Kreissegment des Querschnittsumrisses vo dem zweiten Endabschnitt (328) der hohlen Nähnadel (300) korrespondiert, und wobei eine Schulter (325) von einem Rand (329) der ebenen Fläche (323) aus distal hin zu der zweiten spitzen Spitze (300) hervorsteht.

6. Nähen-Endeffektor (901) gemäß einem von den Ansprüchen 1 bis 5, wobei die erste Backe (100) und die zweite Backe (200) miteinander über ein Schwenken-Gelenk (190) in einer Weise verbunden sind, um eine Schwenken-Bewegung relativ zueinander um eine Schwenken-Achse (914) des Schwenken-Gelenks (190) bereitzustellen derart, dass die erste Backe (100) und die zweite Backe (200) relativ zueinander öffenbar und schließbar sind.

7. Nähen-Endeffektor (901) gemäß einem von den Ansprüchen 1 bis 6, welcher ferner aufweist: eine Gieren-Verbindung (400), welche mit der Backeneinrichtung (902) über ein Gieren-Gelenk (420) in einer Weise gekoppelt ist, um der Backeneinrichtung (902) eine Gieren-Bewegung relativ zu der Gieren-Verbindung (400) um eine Gieren-Achse (912) der Backeneinrichtung herum (902) bereitzustellen, und/oder eine Nicken-Verbindung (500), welche mit der Backeneinrichtung (902) über ein Nicken-Gelenk (520a, 502b) in einer Weise gekoppelt ist, um der Backeneinrichtung (902) eine Nicken-Bewegung relativ zu der Nicken-Verbindung um eine Nicken-Achse (913) der Backeneinrichtung (902) herum bereitzustellen.

8. Nähen-Endeffektor (901) gemäß Anspruch 6 oder 7, wobei das Schwenken-Gelenk (190) und/oder das Gieren-Gelenk (420) und/oder das Nicken-Gelenk (520) jeweils einen Zugelement-und-Umlenkrolle-Mechanismus (704) aufweist, welcher konfiguriert ist, um die jeweils zugeordnete Bewegung zu steuern, wobei der Zugelement-und-Umlenkrolle-Mechanismus (704) eine Umlenkrolle (260, 140, 440), welche drehbar koaxial zu den jeweils zugeordneten Achsen (914, 912, 913) angeordnet ist, und ein Zugelement (750, 751, 730, 731, 720, 721) aufweist, welches um die Umlenkrolle (260, 140, 440) in einer Weise derart geschlungen ist, dass ein Ziehen an einer von beiden Seiten von dem Zugelement (750, 751, 730, 731, 720, 721) mit Bezug auf die Umlenkrolle (260, 140, 440) bewirkt, dass sich die Umlenkrolle (260, 140, 440) in einer jeweils zugeordneten Richtung dreht, um die jeweilige Bewegung der jeweiligen Gelenke zu erzeugen, wobei vorzugsweise die Umlenkrolle (260, 140, 440) ein Halteelement aufweist, und das Zugelement (750, 751, 730, 731, 720, 721) einen Knoten aufweist, welcher in dem Halteelement der Umlenkrolle (260, 140, 440) in einer Weise derart gehalten ist, dass der Knoten in das Halteelement eingreift, um die Umlenkrolle (260, 140, 440) zu drehen, wenn das Zugelement (750, 751, 730, 731, 720, 721) gezogen wird, und wobei vorzugsweise das Halteelement der Umlenkrolle (260, 140, 440) einen ausgesparten Abschnitt aufweist, welcher in einen Bereich der Umlenkrolle (260, 140, 440) hineingeschnitten ist, und der Knoten des Zugelements (750, 751, 730, 731, 720, 721) eine auf das Zugelement (750, 751, 730, 731, 720, 721) gecrimpte Crimp-Perle (712, 722, 732) aufweist.

9. Nähen-Endeffektor (901) gemäß einem von den Ansprüchen 1 bis 8, wobei die Backeneinrichtung (902) ferner einen Zugelement-und-Umlenkrolle-Arretierung-Mechanismus (704) aufweist, welcher konfiguriert ist, um die hohle Nähnadel (300) wahlweise an der ersten Backe (100) und der zweiten Backe (200) zu arretieren,
wobei der Zugelement-und-Umlenkrolle-Arretierung-Mechanismus (704) eine erste Umlenkrolle (120), welche in der ersten Backe (100) angeordnet ist, eine zweite Umlenkrolle (270), welche an einem Gelenk (190) zwischen der ersten Backe (100) und der zweiten Backe (200) angeordnet ist, und eine dritte Umlenkrolle (220) aufweist, welche in der zweiten Backe (200) angeordnet ist,
wobei ein Zugelement (710, 711) um die erste Umlenkrolle (120), die zweite Umlenkrolle (270) und die dritte Umlenkrolle (220) in einer Weise geschlungen ist, in welcher ein erstes Segment (710) von dem Zugelement zwischen der ersten Umlenkrolle (120) und der zweiten Umlenkrolle (270) parallel zu der Eingriffsfläche (116) der ersten Backe (100) ist und ein zweites Segment (711) von dem Zugelement zwischen der zweiten Umlenkrolle (270) und der dritten Umlenkrolle (220) parallel zu der Eingriffsfläche (226) der zweiten Backe (200) ist,
wobei ein erstes Arretierungselement (150) an dem ersten Segment (710) von dem Zugelement angebracht ist und ein zweites Arretierungselement (250) an dem zweiten Segment (711) von dem Zugelement angebracht ist,
wobei ein Ziehen eines ersten Endes von dem Zugelement bewirkt, dass sich das erste Arretierungselement (150) bewegt und sich über mindestens einen Teil von dem Durchgangsloch (110) der ersten Backe (100) legt zum In-Eingriff-Sein mit der hohlen Nähnadel (300), um die hohle Nähnadel (300) an der ersten Backe (100) zu arretieren, und bewirkt, dass sich das zweite Arretierungselement (250) von dem Durchgangsloch (210) der zweiten Backe (200) aus wegbewegt, zum Freigeben der hohlen Nähnadel (300), und
wobei ein Ziehen eines zweiten Endes von dem Zugelement bewirkt, dass sich das zweite Arretierungselement (250) bewegt und sich über mindestens einen Teil von dem Durchgangsloch (210) der zweiten Backe (200) legt zum In-Eingriff-Sein mit der hohlen Nähnadel (300), um die hohle Nähnadel (300) an der zweiten Backe (200) zu arretieren, und bewirkt, dass sich das erste Arretierungselement (150) von dem Durchgangsloch (110) der ersten Backe (100) aus wegbewegt zum Freigeben der hohlen Nähnadel (300).

10. Nähen-Endeffektor gemäß einem von den Ansprüchen 1 bis 8, wobei die Backeneinrichtung ferner einen Zugelement-und-Umlenkrolle-Arretierung-Mechanismus (705) aufweist, welcher konfiguriert ist, um die hohle Nähnadel (300) wahlweise an der ersten Backe (100) und der zweiten Backe (200) zu arretieren,
wobei der Zugelement-und-Umlenkrolle-Arretierung-Mechanismus (705) aufweist:
einen ersten Arretierung-Sub-Mechanismus, welcher eine erste Umlenkrolle (120'), welche in der ersten Backe (100) angeordnet ist, und ein erstes Zugelement (710') aufweist, welches um die erste Umlenkrolle (120') geschlungen ist, und
einen zweiten Arretierung-Sub-Mechanismus, welcher eine zweite Umlenkrolle (220'), welche in der zweiten Backe (200) angeordnet ist, und ein zweites Zugelement (711') aufweist, welches um die zweite Umlenkrolle (220') geschlungen ist,
wobei ein erstes Arretierungselement (150') an dem ersten Zugelement (710') angebracht ist und ein zweites Arretierungselement (250') an dem zweiten Zugelement (711') angebracht ist,
wobei ein Ziehen eines ersten Endes von dem ersten Zugelement (710') bewirkt, dass sich das erste Arretierungselement (150') bewegt und sich über mindestens einem Teil von dem Durchgangsloch (110) der ersten Backe (100) legt zum In-Eingriff-Sein mit der hohlen Nähnadel (300), um die hohle Nähnadel (300) an der ersten Backe (100) zu arretieren, und ein Ziehen eines zweiten Endes von dem ersten Zugelement (710') bewirkt, dass sich das erste Arretierungselement (150') von dem Durchgangsloch (110) der ersten Backe (100) aus wegbewegt zum Freigeben der hohlen Nähnadel (300),
wobei ein Ziehen eines ersten Endes von dem zweiten Zugelement (711') bewirkt, dass sich das zweite Arretierungselement (250') bewegt und sich über mindestens einen Teil von dem Durchgangsloch (210) der zweiten Backe (200) legt zum In-Eingriff-Sein mit der hohlen Nähnadel (300), um die hohle Nähnadel (300) an der zweiten Backe (200) zu arretieren, und ein Ziehen eines zweiten Endes von dem zweiten Zugelement (711') bewirkt, dass sich das zweite Arretierungselement (250') von dem Durchgangsloch (2100) der zweiten Backe (200) aus wegbewegt zum Freigeben der hohlen Nähnadel (300),
wobei der erste Arretierung-Sub-Mechanismus und der zweite Arretierung-Sub-Mechanismus unabhängig voneinander betätigbar sind.

11. Nähen-Endeffektor (901) gemäß Anspruch 9 oder 10, wobei das erste Arretierungselement (150) und das zweite Arretierungselement (250) jeweils ein Arretierungsblatt aufweisen, welches einer Länge nach an einem jeweiligen Segment des jeweils zugeordneten Zugelements angebracht ist, und
wobei die hohle Nähnadel (300) einen ersten Schlitz (320), welcher sich quer über die hohle Nähnadel (300) hinweg erstreckt und in Richtung hin zu der ersten spitzen Spitze (332) angeordnet ist, und einen zweiten Schlitz (321) aufweist, welcher sich quer über die hohle Nähnadel (300) hinweg erstreckt und in Richtung hin zu der zweiten spitzen Spitze (334) angeordnet ist,
wobei die jeweiligen Arretierungsblätter mit der hohle Nähnadel (300) in Eingriff kommen mittels Kreuzens mit dem jeweils zugeordneten von dem ersten Schlitz (320) und dem zweiten Schlitz (321) der hohlen Nähnadel (300), um die hohle Nähnadel (300) an der jeweils zugeordneten von der ersten Backe (100) und der zweiten Backen (200) zu arretieren.

12. Nähen-Endeffektor (901) gemäß einem von den Ansprüchen 9 bis 11, wobei jede
Umlenkrolle (120, 270, 220, 120', 220') des Zugelement-und-Umlenkrolle-Arretierung-Mechanismus (704, 705) eine sich drehende Umlenkrolle oder eine fixierte runde Kontur aufweist.

## Revendications

1. Effecteur terminal de suture (1, 901), comprenant :
un ensemble de mâchoires (2, 902), comprenant
une première mâchoire (10, 100) présentant un trou traversant (11, 110) qui est disposé au niveau d'une partie d'extrémité (14, 114) de la première mâchoire (10, 100) et qui s'étend à travers la première mâchoire (10, 100) depuis une surface de mise en prise (16, 116) de la première mâchoire (10, 100) vers une surface opposée (18, 118) de la première mâchoire (10, 100),
une seconde mâchoire (20, 200) présentant un trou traversant (21, 210) qui est disposé au niveau d'une partie d'extrémité (24, 224) de la seconde mâchoire (20, 200) et qui s'étend à travers la seconde mâchoire (20, 200) depuis une surface de mise en prise (26, 226) de la seconde mâchoire (20, 200) vers une surface opposée (28, 228) de la seconde mâchoire (20, 200),
dans lequel la première mâchoire (10, 100) et la seconde mâchoire (20, 200) peuvent être ouvertes et fermées l'une par rapport à l'autre, la surface de mise en prise (16, 116) de la première mâchoire (10, 100) et la surface de mise en prise (26, 226) de la seconde mâchoire (20, 200) étant dirigées l'une vers l'autre,
**caractérisé en ce que** l'effecteur terminal de suture (1, 901) comprend en outre
un assemblage d'aiguille de suture (3, 903) comprenant une aiguille de suture creuse (30, 300) présentant une première extrémité pointue (32, 332), une seconde extrémité pointue (34, 334) et un canal interne (36, 336) s'étendant à travers l'aiguille de suture creuse (30, 300) de la première extrémité pointue (32, 332) à la seconde extrémité pointue (34, 334),
dans lequel, dans une disposition rangée de l'effecteur terminal de suture (1, 901), l'aiguille de suture creuse (30, 300) est serrée à plat entre la surface de mise en prise (16, 116) de la première mâchoire (10, 100) et la surface de mise en prise (26, 226) de la seconde mâchoire (20, 200), et un fil guide (76, 760) s'étend à travers le trou traversant (11, 110) de la première mâchoire (10, 100), le canal interne (36, 336) de l'aiguille de suture creuse (30, 300) et le trou traversant (21, 210) de la seconde mâchoire (20, 200) de telle manière que l'ouverture de la première mâchoire (10, 100) et de la seconde mâchoire (20, 200) l'une par rapport à l'autre et la mise en tension du fil guide (76, 760) amènent le fil guide (76, 760) à aligner l'aiguille de suture creuse (30, 300) avec la première extrémité pointue (32, 332) de l'aiguille de suture creuse (30, 300) dirigée vers le trou traversant (11, 110) de la première mâchoire (10, 100) et la seconde extrémité pointue (34, 334) de l'aiguille de suture creuse (30, 300) dirigée vers le trou traversant (21, 210) de la seconde mâchoire (20, 200).

2. Effecteur terminal de suture (901) selon la revendication 1, dans lequel l'aiguille de suture creuse (300) présente une ouverture (330) le long de l'aiguille de suture creuse (300) entre la première extrémité pointue (332) et la seconde extrémité pointue (334), l'ouverture (330) fournissant un accès au canal interne (336) de l'aiguille de suture creuse (300), et dans lequel l'ensemble à aiguille de suture (903) comprend en outre un fil de suture (340) couplé à l'ouverture (330) le long de l'aiguille de suture creuse (300).

3. Effecteur terminal de suture (901) selon la revendication 2, dans lequel une extrémité du fil de suture (340) est insérée à travers l'ouverture (330) le long de l'aiguille de suture creuse (300) jusque dans l'aiguille de suture creuse (300), et dans lequel l'ensemble à aiguille de suture (300) comprend en outre un tube (350) inséré à travers le canal interne (336) de l'aiguille de suture creuse (300) de manière à coincer l'extrémité insérée du fil de suture (340) entre une surface intérieure du canal interne (336) de l'aiguille de suture creuse (300) et une surface extérieure du tube (350) de manière à fixer le fil de suture (340) à l'aiguille de suture creuse (300), et dans lequel le fil guide (760) s'étend à travers un canal interne (356) du tube (350).

4. Effecteur terminal de suture (901) selon l'une quelconque des revendications 1 à 3, dans lequel un contour de section transversale d'une première section d'extrémité (326) de l'aiguille de suture creuse (300) vers la première extrémité pointue (332) est d'une forme sans symétrie de rotation et un contour de section transversale du trou traversant (110) de la première mâchoire (100) est d'une forme correspondante de telle sorte que la première section d'extrémité (326) de l'aiguille de suture creuse (300) s'ajuste dans le trou traversant (110) de la première mâchoire (100) dans une orientation de l'aiguille de suture creuse (300) par rapport au trou traversant (110) de la première mâchoire (100), de préférence dans lequel le contour de section transversale de la première section d'extrémité (326) de l'aiguille de suture creuse (300) est d'une forme ressemblant à un cercle avec un segment circulaire découpé, et de préférence dans lequel la première section d'extrémité (326) de l'aiguille de suture creuse (300) comprend une surface plate (322) correspondant au segment circulaire découpé du contour de section transversale de la première section d'extrémité (326) de l'aiguille de suture creuse (300), et dans lequel un épaulement (324) fait saillie à partir d'un bord (327) de la surface plate (322) distal par rapport à la première extrémité pointue (332).

5. Effecteur terminal de suture (901) selon l'une quelconque des revendications 1 à 4, dans lequel un contour de section transversale d'une seconde section d'extrémité (328) de l'aiguille de suture creuse (300) vers la seconde extrémité pointue (334) est d'une forme sans symétrie de rotation et un contour de section transversale du trou traversant (210) de la seconde mâchoire (200) est d'une forme correspondante de telle sorte que la seconde section d'extrémité (328) de l'aiguille de suture creuse (300) s'ajuste dans le trou traversant (210) de la seconde mâchoire (200) dans une orientation de l'aiguille de suture creuse (300) par rapport au trou traversant (210) de la seconde mâchoire (200), de préférence dans lequel le contour de section transversale de la seconde section d'extrémité (328)de l'aiguille de suture creuse (300) est d'une forme ressemblant à un cercle avec un segment circulaire découpé, et de préférence, dans lequel la seconde section d'extrémité (328) de l'aiguille de suture creuse (300) comprend une surface plate (323) correspondant au segment circulaire découpé du contour de section transversale de la seconde section d'extrémité (328) de l'aiguille de suture creuse (300), et dans lequel un épaulement (325) fait saillie à partir d'un bord (329) de la surface plate (323) distal par rapport à la seconde extrémité pointue (300).

6. Effecteur terminal de suture (901) selon l'une quelconque des revendications 1 à 5, dans lequel la première mâchoire (100) et la seconde mâchoire (200) sont reliées l'une à l'autre par l'intermédiaire d'une articulation pivotante (190) de manière à fournir un mouvement de pivotement l'une par rapport à l'autre autour d'un axe de pivotement (914) de l'articulation pivotante (190) de sorte que la première mâchoire (100) et la seconde mâchoire (200) peuvent être ouvertes et fermées l'une par rapport à l'autre.

7. Effecteur terminal de suture (901) selon l'une quelconque des revendications 1 à 6, comprenant en outre une liaison de lacet (400) couplée à l'ensemble de mâchoires (902) via une articulation de lacet (420) de manière à fournir un mouvement de lacet à l'ensemble de mâchoires (902) par rapport à la liaison de lacet (400) autour d'un axe de lacet (912) de l'ensemble de mâchoires (902), et/ou une liaison de pas (500) couplée à l'ensemble de mâchoires (902) via une articulation de pas (520a, 502b) de manière à fournir un mouvement de pas à l'ensemble de mâchoires (902) par rapport à la liaison de pas autour d'un axe de pas (913) de l'ensemble de mâchoires (902).

8. Effecteur terminal de suture (901) selon la revendication 6 ou 7, dans lequel l'articulation pivotante (190) et/ou l'articulation de lacet (420) et/ou l'articulation de pas (520) comprennent chacune un mécanisme de tendon et de poulie (704) configuré pour commander le mouvement respectif, dans lequel le mécanisme de tendon et de poulie (704) comprend une poulie (260, 140, 440) disposée de manière rotative coaxialement par rapport aux axes respectifs (914, 912, 913) et un tendon (750, 751, 730, 731, 720, 721) enroulé autour de la poulie (260, 140, 440) de telle sorte qu'une traction de part et d'autre du tendon (750, 751, 730, 731, 720, 721) par rapport à la poulie (260, 140, 440) amène la poulie (260, 140, 440) à tourner dans une direction respective de manière à générer le mouvement respectif des articulations respectives, de préférence dans lequel la poulie (260, 140, 440) comprend un élément de retenue et le tendon (750, 751, 730, 731, 720, 721) comprend un noeud retenu dans l'élément de retenue de la poulie (260, 140, 440) de telle sorte que le noeud met en prise l'élément de retenue pour faire tourner la poulie (260, 140, 440) lorsque le tendon (750, 751, 730, 731, 720, 721) est tiré, et de préférence dans lequel l'élément de retenue de la poulie (260, 140, 440) comprend une partie évidée découpée dans un secteur de la poulie (260, 140, 440) et le noeud du tendon (750, 751, 730, 731, 720, 721) comprend un bourrelet de sertissage (712, 722, 732) serti sur le tendon (750, 751, 730, 731, 720, 721).

9. Effecteur terminal de suture (901) selon l'une quelconque des revendications 1 à 8, dans lequel l'ensemble de mâchoires (902) comprend en outre un mécanisme de verrouillage de tendon et de poulie (704) configuré afin de verrouiller alternativement l'aiguille de suture creuse (300) sur la première mâchoire (100) et la seconde mâchoire (200),
dans lequel le mécanisme de verrouillage de tendon et de poulie (704) comprend une première poulie (120) disposée dans la première mâchoire (100), une deuxième poulie (270) disposée au niveau d'une articulation (190) entre la première mâchoire (100) et la seconde mâchoire (200), et une troisième poulie (220) disposée dans la seconde mâchoire (200),
dans lequel un tendon (710, 711) est enroulé autour de la première poulie (120), de la deuxième poulie (270) et de la troisième poulie (220) d'une manière telle qu'un premier segment (710) du tendon entre la première poulie (120) et la deuxième poulie (270) est parallèle à la surface de mise en prise (116) de la première mâchoire (100) et qu'un second segment (711) du tendon entre la deuxième poulie (270) et la troisième poulie (220) est parallèle à la surface de mise en prise (226) de la seconde mâchoire (200),
dans lequel un premier élément de verrouillage (150) est fixé au premier segment (710) du tendon et un second élément de verrouillage (250) est fixé au second segment (711) du tendon,
dans lequel une traction d'une première extrémité du tendon amène le premier élément de verrouillage (150) à se déplacer et à se trouver à travers au moins une partie du trou traversant (110) de la première mâchoire (100) pour mettre en prise l'aiguille de suture creuse (300) afin de verrouiller l'aiguille de suture creuse (300) à la première mâchoire (100), et amène le second élément de verrouillage (250) à s'éloigner du trou traversant (210) de la seconde mâchoire (200) pour libérer l'aiguille de suture creuse (300), et
dans lequel la traction d'une seconde extrémité du tendon amène le second élément de verrouillage (250) à se déplacer et à se trouver à travers au moins une partie du trou traversant (210) de la seconde mâchoire (200) pour mettre en prise l'aiguille de suture creuse (300) afin de verrouiller l'aiguille de suture creuse (300) à la seconde mâchoire (200), et amène le premier élément de verrouillage (150) à s'éloigner du trou traversant (110) de la première mâchoire (100) pour libérer l'aiguille de suture creuse (300).

10. Effecteur terminal de suture selon l'une quelconque des revendications 1 à 8, dans lequel l'ensemble de mâchoires comprend en outre un mécanisme de verrouillage de tendon et de poulie (705) configuré afin de verrouiller alternativement l'aiguille de suture creuse (300) sur la première mâchoire (100) et la seconde mâchoire (200),
dans lequel le mécanisme de verrouillage de tendon et de poulie (705) comprend
un premier sous-mécanisme de verrouillage comprenant une première poulie (120') disposée dans la première mâchoire (100) et un premier tendon (710') enroulé autour de la première poulie (120'),
un second sous-mécanisme de verrouillage comprenant une deuxième poulie (220') disposée dans la seconde mâchoire (200) et un second tendon (711') enroulé autour de la deuxième poulie (220'),
dans lequel un premier élément de verrouillage (150') est fixé au premier tendon (710') et un second élément de verrouillage (250') est fixé au second tendon (711'),
dans lequel une traction d'une première extrémité du premier tendon (710') amène le premier élément de verrouillage (150') à se déplacer et à se trouver à travers au moins une partie du trou traversant (110) de la première mâchoire (100) pour mettre en prise l'aiguille de suture creuse (300) afin de verrouiller l'aiguille de suture creuse (300) à la première mâchoire (100), et une traction d'une seconde extrémité du premier tendon (710') amène le premier élément de verrouillage (150') à s'éloigner du trou traversant (110) de la première mâchoire (100) pour libérer l'aiguille de suture creuse (300),
dans lequel une traction d'une première extrémité du second tendon (711') amène le second élément de verrouillage (250') à se déplacer et à se trouver à travers au moins une partie du trou traversant (210) de la seconde mâchoire (200) pour mettre en prise l'aiguille de suture creuse (300) afin de verrouiller l'aiguille de suture creuse (300) à la seconde mâchoire (200), et une traction d'une seconde extrémité du second tendon (711') amène le second élément de verrouillage (250') à s'éloigner du trou traversant (2100) de la seconde mâchoire (200) pour libérer l'aiguille de suture creuse (300),
dans lequel le premier sous-mécanisme de verrouillage et le second sous-mécanisme de verrouillage peuvent fonctionner indépendamment.

11. Effecteur terminal de suture (901) selon la revendication 9 ou 10,
dans lequel le premier élément de verrouillage (150) et le second élément de verrouillage (250) comprennent chacun une lame de verrouillage attachée longitudinalement au segment respectif du tendon respectif, et
dans lequel l'aiguille de suture creuse (300) comprend une première fente (320) à travers l'aiguille de suture creuse (300) et disposée vers la première extrémité pointue (332), et une seconde fente (321) à travers l'aiguille de suture creuse (300) et disposée vers la seconde extrémité pointue (334),
dans lequel des lames de verrouillage respectives mettent en prise l'aiguille de suture creuse (300) en croisant les première et seconde fentes respectives (320, 321) de l'aiguille de suture creuse (300) afin de verrouiller l'aiguille de suture creuse (300) aux première et seconde mâchoires respectives (100, 200).

12. Effecteur terminal de suture (901) selon l'une quelconque des revendications 9 à 11, dans lequel chaque poulie (120, 270, 220, 120', 220') du mécanisme de verrouillage de tendon et de poulie (704, 705) comprend une poulie rotative ou un contour rond fixe.
